# EUROPEAN PATENT APPLICATION

(11) **EP 4 520 823 A1**
(43) Date of publication of application: **12.03.2025**
(21) Application number: 23799682.2
(22) Date of filing: 03.05.2023
(51) Int. Cl.: C12N 15/11, C12N 15/63, C12N 15/67

(54) **UTR SEQUENCE FOR CONTROLLING PROTEIN EXPRESSION LEVEL AND EXPRESSION LOCATION, AND MRNA SEQUENCE INCLUDING SAME**

(30) Priority: 03.05.2022 KR 20220055103; 01.11.2022 KR 20220144066
(71) Applicant: Industry-Academic Cooperation Foundation Dankook University, Yongin-si, Gyeonggi-do 16890 (KR)
(72) Inventor: JEONG, Sunjoo, Seongnam-si, Gyeonggi-do 13531 (KR); KIM, Narae, Seoul 05078 (KR); JEONG, Jiwon, Yongin-si, Gyeonggi-do 16987 (KR); HONG, Dawon, Suwon-si, Gyeonggi-do 16695 (KR)
(74) Representative: J A Kemp LLP
(86) International application number: PCT/KR2023/006052
(87) International publication number: WO 2023/214801

(57) **Abstract**

The present invention relates to an mRNA including the UTR of polymorphic β-catenin. When used, the mRNA molecule, nucleic acid molecule, expression construct, and/or expression vector of the present invention can effectively enhance the expression efficiency of a target protein and extend the expression location of the target protein to the cytoplasm, thus allowing for stable extracellular secretion. In addition, the mRNA functions to regulate an expression level of exogenously introduced mRNA and thus can be utilized as an mRNA vaccine in the future.

## Description

### Technical Field

The present invention was developed with the support of the Ministry of Science and ICT of the Republic of Korea, under Project No. 00208597, which was conducted in the research project named "Regulatory network of cellular mRNP localization by Multi-UTR" in the research program titled "Personal Basic Research," by Dankook University Industry-Academia Cooperation, under the management of the National Research Foundation of Korea, from March 1, 2023 to February 29, 2024.

The present invention was additionally developed with the support from the Ministry of Science and ICT of the Republic of Korea, under Project No. 2022M3E5F1016546, which was conducted in the research project named "Development of mRNA vaccine platform technology based on Alternative Spliced UTR (AS-UTR)" in the research program titled "Basic Core Technology Development Project for Next-Generation Vaccines for Infectious Diseases" by Dankook University, under the management of the National Research Foundation of Korea, from April 1, 2022 to December 31, 2022.

This application claims priority to, and the benefit of, Korean Patent Application No. 10-2022-0055103, filed with the Korean Intellectual Property Office on May 3, 2022, the disclosure of which is incorporated herein by reference.

This application also claims priority to, and the benefit of, Korean Patent Application No. 10-2022-0144066, filed with the Korean Intellectual Property Office on November 1, 2022, the disclosure of which is incorporated herein by reference.

The present invention relates to a UTR sequence designed to regulate protein expression level and localization, as well as to an mRNA sequence containing the same.

### Background Art

Messenger RNA (mRNA) serves as a "blueprint" for protein production by RNA molecules. mRNA is transcribed from a DNA template and essentially comprises a coding region that encodes a protein to be synthesized; it may also comprise untranslated regions (UTRs). Untranslated regions are sequences located upstream of the start codon and downstream of the stop codon of mRNA, respectively, and are not translated into a protein. Typical eukaryotic mRNA has UTRs at both the 5' and 3' ends. UTRs significantly affect mRNA stability and expression by regulating mRNA degradation or translation, ultimately playing a crucial role in regulating protein levels. Especially, the 3'UTR contributes to the regulation of mRNA expression and mRNA stability, as well as the intercellular localization of mRNA. Several types of 3'UTR isoforms may be generated from a single gene by alternative splicing, but only a small number of genes correspond to such a group. There has been no report on the effect of each multi-UTR isoform produced from one gene on improving expression efficiency.

Meanwhile, many studies have revealed components of the Wnt signaling pathway and signaling processes. Dr. Hans Clevers' research team at the Hubrecht Institute, which plays a leading role in the field of Wnt signaling, published the Canonical Wnt pathway, and thus, β-catenin, a key regulator, has received more attention. β-catenin, a multi-functional molecule that performs various cellular functions, is a protein expressed from the human CTNNB1 gene (Tomas Valenta and Basler, 2012). β-catenin may have three types of 3'UTRs with different lengths. There have been no prior studies on changes in protein expression patterns and localization based on the 3'UTR isoform of β-catenin mRNA.

The present inventors conducted research on the protein expression efficiency and localization based on the UTR type of β-catenin mRNA. Furthermore, the present inventors conducted research to investigate elements that significantly affect the regulation of expression efficiency and expression localization among 3'UTR isoforms by fragmenting the 3'UTR isoforms of β-catenin mRNA. Therefore, the present inventors propose an mRNA platform system capable of improving the expression efficiency of a target protein and promoting the movement of the target protein to the cytoplasm or cell membrane, enabling stable secretion of the target protein from the cell.

Throughout the specification, numerous papers and patent documents are referenced, and their citations are provided. The disclosures of cited papers and patent documents are entirely incorporated by reference herein, and the level of the technical field within which the present invention falls and details of the present invention are explained more clearly.

### Disclosure of Invention

### Technical Problem

The present inventors have made intensive efforts to develop an mRNA platform system capable of increasing the translation efficiency of mRNA to improve the expression efficiency of a target protein and promoting the movement of the target protein into the cytoplasm or cellular membrane, allowing stable secretion of the target protein outside the cell. As a result, the present inventors established that an mRNA sequence comprising 5'UTR and a specific 3'UTR fragment of β-catenin mRNA showed a significant increase in translation efficiency, thereby ensuring an increase in the rate at which a protein is expressed in both the cytoplasm and cell membrane, thus completed the present invention.

Accordingly, an aspect of the present invention is to provide an mRNA molecule for improving the expression efficiency of a target protein and promoting the movement of the target protein into the cytoplasm or cellular membrane.

Another aspect of the present invention is to provide a nucleic acid molecule encoding the mRNA molecule.

Still, another aspect of the present invention is to provide an expression construct comprising the nucleic acid molecule.

Still, another aspect of the present invention is to provide a recombinant vector comprising the expression construct.

Still, another aspect of the present invention is to provide an isolated host cell comprising the expression vector.

Still, another aspect of the present invention is to provide an mRNA preparation method capable of enhancing the expression efficiency of the target protein and promoting the movement of the target protein into a cytoplasm or a cellular membrane.

Other purposes and advantages of the present disclosure will become more obvious when taken with the following detailed description of the invention, claims, and drawings.

### Solution to Problem

In accordance with one aspect of the present invention, there is provided an mRNA molecule comprising (a) a coding region encoding a target protein, (b) a 5'untranslated region (UTR) of β-catenin attached to the 5'-end of the coding region, and (c) a 3'UTR of β-catenin or a fragment thereof attached to the 3'-end of the coding region.

The present inventors have made intensive efforts to develop an mRNA platform system capable of enhancing the translation efficiency of mRNA to improve the expression efficiency of a target protein and promote the movement of the target protein into the cytoplasm or cellular membrane, allowing the stable secretion of the target protein outside the cell. As a result, the present inventors established that an mRNA sequence comprising5'UTR and a specific 3'UTR fragment of β-catenin mRNA showed a significant increase in translation efficiency, thereby enhancing the rate at which a protein is expressed within the cytoplasm or cell membrane

As used herein, the term "β-catenin (beta-catenin)", also known as Catenin beta-1, refers to a protein expressed from the human CTNNB1 gene. β-catenin is a subunit of the cadherin protein complex and is known as an important factor in Wnt cell signaling, involved in cell fate determination, cell movement, and cell polarity regulation. The human CTNNB1 gene is accessible through a known database, such as GenBank (CTNNB1, catenin beta 1 [Homo sapiens (human)], Gene ID: 1499).

As used herein, the term "target protein" refers to a protein that a person skilled in the art attempts to mass-produce by improving the expression efficiency through the mRNA molecule of the present invention or to secrete stably outside the cell by promoting its movement into the cytoplasm or cellular membrane. The target protein comprises any protein, including β-catenin. In an embodiment of the present invention, the target protein may be a protein other than β-catenin.

As used herein, the term "coding region encoding a target protein" refers to a part of mRNA that consists of codons. The codons are translated into a protein according to the information provided by the genetic code. The coding region typically begins with a start codon and ends with a stop codon. Generally, the start codon is the AUG triplet, and the stop codon is UAA, UAG, or UGA. In addition to coding for protein synthesis, parts of the coding region may act as regulatory sequences in pre-mRNA, functioning as exonic splicing enhancers or exonic splicing silencers. The coding region of the present invention encoding the target protein may comprise not only a coding region sequence of the β-catenin gene but also a sequence transcribed from nucleic acid sequences of various exogenous genes excluding the β-catenin gene. This region may comprise a coding region having mutations in various nucleotides even though it is an mRNA sequence for obtaining the same target protein since there are mutations in nucleotides that cause no protein change. The foregoing mutations in nucleotides that cause no protein change include functionally equivalent codons or codons encoding the same amino acid (for example, due to codon degeneracy, there are six codons for arginine or serine) or codons encoding biologically equivalent amino acids. Considering the foregoing mutations having biologically equivalent activity, it is construed that the nucleic acid molecule used in the present invention also includes a sequence showing substantial identity with the sequence described in the sequence listing. An mRNA molecule in which 5'UTR and 3'UTR or a fragment thereof of the present invention are attached to a coding region of an mRNA sequence capable of translating a protein to be introduced into a subject can be used to significantly increase the translation level of the protein, thereby improving the expression efficiency, and promote the movement of the protein into the cytoplasm or cell membrane, thereby allowing the protein to be stably secreted to the outside of the cell.

As used herein, the term "untranslated region" or "UTR" refers to an untranslated sequence located upstream of the start codon or downstream of the stop codon of mRNA. The UTR upstream of the start codon of mRNA is called 5'UTR, while the UTR downstream of the stop codon of mRNA is called 3'UTR. The untranslated regions in mRNA play a crucial role in regulating both mRNA stability and mRNA translation. Through the functional characteristics of UTRs, the present inventors have improved the expression efficiency of a target protein by increasing the translation efficiency of β-catenin mRNA, which had not been studied in the conventional art, and allows the target protein to be stably secreted outside the cell by promoting its movement into the cytoplasm or cellular membrane.

As used herein, the term "5'UTR of β-catenin" refers to 5'UTR of mRNA of the β-catenin gene, and the term "3'UTR of β-catenin" refers to 3'UTR of mRNA of the β-catenin gene.

The term "3'UTR of β-catenin" or "fragment of 3'UTR of β-catenin" refers to a type of 3'UTR sequence that was verified for their translation efficiency for the optimization of the 3'UTR sequence of mRNA of the β-catenin gene by the present inventors, and may comprise isoforms by alternative splicing of 3'UTR and fragments (F1, F2, F3, F4 or a combination thereof) of 3'UTR fragmented in the examples to be described later. β-catenin has three types of 3'UTR isoforms (UTR-1, UTR-2, and UTR-3) by alternative splicing, although it has the same coding region. UTR-3 is the longest isoform, comprising intron 15 (In15) and exon 16 (E16). UTR-2 is an intermediate-length isoform with intron 15 removed. UTR-1 is the shortest isoform with intron 15 (In15) and exon 16A (E16A) removed. The translation efficiency of mRNA and the distribution localization of an expressed protein may vary depending on 3'UTR or the fragment thereof.

In an embodiment of the present invention, the coding region encoding the target protein of the mRNA molecule of the present invention may comprise a start codon.

In an embodiment of the present invention, the coding region encoding the target protein of the mRNA molecule of the present invention may comprise a stop codon.

In an embodiment of the present invention, the fragment of 3'UTR of β-catenin of the present invention has a deletion of at least an intron 15 (In15) in the full-length 3'UTR sequence. In the present invention, deletion of an intron 15 (In15) encompasses a deletion of all or part of the intron 15 site.

As used herein, the term "intron 15 (In15) site" is set forth in SEQ ID NO: 3 and refers to the nucleotide sequence from the 13th to the 317th positions in SEQ ID NO: 8. The nucleotide sequence of SEQ ID NO: 8 herein is an isoform comprising an In15 site and is referred to as UTR-3 herein. mRNA sequences comprising 3'UTR sequences comprising an In15 site show restricted movement into the cytoplasm or cell membrane, while mRNA sequences comprising 3'UTR sequences lacking an In15 site facilitate movement into the cytoplasm, consequently impacting protein translation efficiency.

In an embodiment of the present invention, the fragment of the 3'UTR of β-catenin of the present invention further has a deletion of an exon 16A (E16A) site. In the present invention, the deletion of an exon 16A site encompasses the removal of all or part of the exon 16A site. As used herein, the "exon 16A (E16A) site" is set forth in SEQ ID NO: 4 and refers to the nucleotide sequence from the 13th to 171st positions in SEQ ID NO: 7 and from the 318th to 476th positions in SEQ ID NO: 8. Especially, as validated in examples described later, when 5'UTR and 3'UTR of β-catenin are used together, the use of 3'UTR with a deletion of an In15 site and inclusion of an E16A site enhances the expression efficiency of a protein encoded by the coding region.

In an embodiment of the present invention, the fragment of 3'UTR of β-catenin of the present invention may comprise an RNA sequence selected from the group consisting of the RNA sequence of SEQ ID NO: 6, the RNA sequence of SEQ ID NO: 7, the RNA sequence of SEQ ID NO: 9, the RNA sequence of SEQ ID NO: 10, the RNA sequence of SEQ ID NO: 11, the RNA sequence of SEQ ID NO: 12, the RNA sequence of SEQ ID NO: 13, the RNA sequence of SEQ ID NO: 14, the RNA sequence of SEQ ID NO: 15, and a combination thereof. More specifically, the fragment of 3'UTR of β-catenin comprises an RNA sequence selected from the group consisting of the RNA sequence of SEQ ID NO: 9, the RNA sequence of SEQ ID NO: 11, the RNA sequence of SEQ ID NO: 13, and a combination thereof, and more specifically, comprises the RNA sequence of SEQ ID NO: 13.

Herein, the "promoting the movement of a target protein into the cytoplasm or cell membrane" means that the proportion of a target protein localized to the cytoplasm or cell membrane increases, or the distribution range of the expressed target protein localized to the cytoplasm is further away from the nucleus. As validated in examples described later, the presence of 5'UTR together with F1+F3 (SEQ ID NO: 13) as a 3'UTR fragment in β-catenin increased the proportion of the expression localization of a target protein observed in the cytoplasm and cell membrane, which are away from the nucleus, thereby exhibiting a distinct effect of regulating the expression localization of the target protein so as to enable the protein to be stably secreted to the outside of the cells.

The 5'UTR, 3'UTR, and 3'UTR fragments of β-catenin used in the present invention are construed to encompass sequences showing substantial identity to the sequences set forth in SEQ ID NO: 1 and SEQ ID NOs: 6 to 15. The substantial identity denotes at least 60% homology (e.g., 60%, 61%, 62%, 63%, 64%, 65%, 66%, 67%, 68%, or 69%), more preferably at least 70% homology (e.g., 70%, 71%, 72%, 73%, 74%, 75%, 76%, 77%, 78%, or 79%), still more preferably at least 80% homology (e.g., 80%, 81%, 82%, 83%, 84%, 85%, 86%, 87%, 88%, or 89%), and most preferably at least 90% homology (e.g., 90%, 91%, 92%, 93%, 94%, 95%, 96%, 97%, 98%, 99%, or 100%) in sequences when the sequences of the present invention are aligned with any other sequence so as to match each other as much as possible and the aligned sequences are analyzed using an algorithm commonly used in the art. All integers of 70% to 100% and prime numbers between are included within the scope of the present invention with respect to % homology.

Methods of the alignment for sequence comparison are known in the art. Various methods and algorithms for alignment are disclosed in Smith and Waterman, Adv. Appl. Math. 2:482(1981) Needleman and Wunsch, J. Mol. Bio. 48:443(1970); Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992); and Pearson et al., Meth. Mol. Biol. 24:307-31(1994). NCBI Basic Local Alignment Search Tool (BLAST) (Altschul et al., J. Mol. Biol. 215:403-10 (1990)) is accessible from the National Center for Biological Information (NCBI) and is available in conjunction with sequencing programs such as blastp, blastn, blastx, tblastn and tblastx on the Internet. BLAST is accessible through the BLAST page on the NCBI website. Sequence homology comparison methods using this program can be found on the BLAST help page of the NCBI website.

The mRNA molecule of the present invention may comprise a 5' cap structure at the 5'-end of 5'UTR and may further comprise a poly(A) tail structure at the 3'-end of 3'UTR. Consequently, the stability and translation efficiency of the mRNA molecule can be further enhanced. Additionally, modified natural nucleotides and artificial nucleotides, such as N1-methyl pseudouridine, which are known to enhance mRNA stability and increase mRNA translation, may be incorporated, and it would be obvious to a person skilled in the art that the modifications discussed above are allowed for the mRNA molecule of the present invention.

The mRNA molecule of the present invention may be used in various manners, such as by direct injection into the body of a subject or by application to *in vitro* cells, and may be utilized for various purposes, such as therapeutic agents utilizing mRNA sequences, mRNA vaccines, disease diagnosis, and drug screening. The mRNA molecule of the present invention can retain improved stability by using various methods known in the art, and specifically, for example, may be used for intracellular delivery by enclosing it in phospholipid membranes.

In an embodiment of the present invention, 5'UTR of the present invention comprises the RNA sequence of SEQ ID NO: 1.

In accordance with another aspect of the present invention, a nucleic acid molecule encoding the mRNA molecule is provided.

As used herein, the term "nucleic acid" is intended to comprehensively include DNA (gDNA and cDNA) and RNA molecules, and the nucleotides as basic constituent units in the nucleic acid molecule include naturally occurring nucleotides, as well as analogs with modified sugar or base moieties (Scheit, Nucleotide Analogs, John Wiley, New York (1980); and Uhlman and Peyman, Chemical Reviews, 90:543-584(1990)).

As used herein, the term "nucleic acid molecule encoding an mRNA molecule" is a nucleic acid molecule serving as a transcription template for the mRNA molecule of the present invention, and it would be obvious to a person skilled in the art that the nucleic acid molecule is not limited to a specific nucleotide sequence.

Herein, the open reading frame (ORF) region of the nucleic acid molecule encoding the mRNA molecule of the present invention may be a sequence transcribed from an exogenous nucleic acid sequence. Since the ORF region has mutations in nucleotides that cause no protein change, even nucleic acid sequences for obtaining the same target protein may include ORF having mutations in various nucleotides. The foregoing mutations in nucleotides that cause no protein change include functionally equivalent codons or codons encoding the same amino acid (for example, due to codon degeneracy, there are six codons for arginine or serine) or codons encoding biologically equivalent amino acids. Considering the foregoing mutations having biologically equivalent activity, it is construed that the nucleic acid molecule used in the present invention also includes a sequence showing substantial identity with the sequence described in the sequence listing. 5'UTR and 3' UTR or fragments thereof correspond to untranslated regions and may have comparatively restricted modifications compared with the ORF region but may allow fine modifications that induce no significant changes in the translation activity at the level of common sense of a person skilled in the art.

In accordance with another aspect of the present invention, an expression construct comprising the nucleic acid molecule is provided.

As used herein, the term "expression construct" is defined to refer to a nucleic acid molecule comprising the minimum elements required for protein expression in a cell. The expression construct of the present invention may include various promoters known in the art. The promoter is operatively linked to the nucleic acid sequence of the present invention to regulate the transcription and/or translation of the nucleic acid sequence.

As used herein, the wording "operatively linked" refers to a functional linkage between a nucleic acid expression control sequence, such as a promoter, a signal sequence, or an array of transcription factor binding sites, and another nucleic acid sequence, whereby the control sequence directs the transcription and/or translation of the other nucleic acid sequence.

In accordance with another aspect of the present invention, a recombinant vector comprising the expression construct is provided.

In accordance with another aspect of the present invention, an isolated host cell comprising the recombinant vector is provided.

The vector system of the present invention may be constructed by various methods known in the art, and a specific method thereof is disclosed in Sambrook et al., Cold Spring Harbor Laboratory Press (2001), which is incorporated herein by reference.

The vector of the present invention may be typically constructed as a vector for cloning or a vector for expression. The vector of the present invention may be constructed by using a prokaryotic or eukaryotic cell as a host.

For example, when the vector of the present invention is an expression vector, and a prokaryotic cell is used as a host, the vector generally includes a strong promoter capable of initiating transcription (e.g., pL^{λ} promoter, trp promoter, lac promoter, T7 promoter, tac promoter, etc.), a ribosome binding site for the initiation of translation, and a transcriptional/translational termination sequence. When *E. coli* is used as a host cell, promoter and operator sites on the *E. coli* tryptophan biosynthesis pathway (Yanofsky, C., J. Bacteriol., 158:1018-1024(1984)) and the leftward promoter from phage λ (pLλ promoter, Herskowitz, I. and Hagen, D., Ann. Rev. Genet., 14:399-445(1980)) may be used as a control site.

The vector usable in the present invention may be constructed by engineering plasmids (e.g., pGL3/Luc, pBABE, pSK349, pSC101, ColE1, pBR322, pUC8/9, pHC79, pGEX series, pET series, and pUC19), phages (e.g., λgt·λ4B, λ-Charon, λΔz1, and M13), or viruses (e.g., SV40, etc.).

In an embodiment of the present invention, the vector may additionally transport a gene encoding a reporter molecule, for example, luciferase and β-glucuronidase.

In a specific embodiment of the present invention, the vector may be a FLAG vector, a YFP vector, or a GFP vector but is not limited thereto.

The host cell of the present invention comprises not only a cell capable of temporarily expressing the vector of the present invention by temporary transformation or transfection with the vector of the present invention but also a stable cell line capable of stably and continuously expressing the vector of the present invention. The term "stable cell line" refers to a cell line where a specific gene is integrated into the genome and is continuously inherited and expressed over multiple generations without loss, even during cell division and proliferation.

A host cell or cell line capable of stably and continuously cloning and expressing the vector of the present invention may employ any host cell that is known in the art, and examples thereof may include *E. coli* strains, such as *E. coli* Origami2, *E. coli* JM109, *E. coli* BL21(DE3), *E. coli* RR1, *E. coli* LE392, *E. coli B, E. coli* X 1776, and *E. coli* W3110, *Bacillus* strains, such as *Bacillus subtilis* and *Bacillus thuringiensis,* and Enterobacteriaceae strains, such as *Salmonella typhimurium, Serratia marcescens,* and various *Pseudomonas* species.

When the vector of the present invention is introduced into eukaryotic cells by transformation or transfection, host cells such as yeast (*Saccharomyce cerevisiae*), insect cells, and animal cells (e.g., HCT116, HT-29, SW480, HEK293, U2OS, CHO (Chinese hamster ovary), W138, BHK, COS-7, HepG2, 3T3, RIN, and MDCK cell lines) may be used.

Herein, the term "transformation" or "transfection" refers to the process of introducing a gene into a host cell to enable its expression. A method for transforming or transfecting a vector with a target gene introduced there into a host cell includes any method for introducing nucleotides into the cell, which may be performed by selecting an appropriate standard technique as known in the art.

When the host cell is a prokaryotic cell, a method for delivering the vector of the present invention into a host cell may be conducted by the CaCl2 method (Cohen, S. N. et al., Proc. Natl. Acac. Sci. USA, 9:2110-2114 (1973)), the Hanahan method (Cohen, S. N. et al., Proc. Natl. Acac. Sci. USA, 9:2110-2114(1973); and Hanahan, D., J. Mol. Biol., 166:557-580(1983)), and the electroporation method Dower, W. J. et al., Nucleic. Acids Res., 16:6127-6145(1988)). When the host cell is eukaryotic, the vector may be injected into a host cell by micro-injection (Capecchi, M. R., Cell, 22:479(1980)), calcium phosphate precipitation (Graham, F. L. et al., Virology, 52:456(1973)), electroporation (Neumann, E. et al., EMBO J., 1:841(1982)), liposome-mediated transfection (Wong, T. K. et al., Gene, 10:87(1980)), DEAE-dextran treatment (Gopal, Mol. Cell Biol., 5:1188-1190(1985)), or gene bombardment (Yang et al., Proc. Natl. Acad. Sci., 87:9568-9572(1990)), or the like.

In accordance with another aspect of the present invention, there is provided a method for preparing an mRNA molecule to improve the expression efficiency of a target protein and promote the movement of the target protein into the cytoplasm or cell membrane. The method comprises attaching a 5'-UTR of β-catenin and a 3'UTR of β-catenin or a fragment thereof to the 5'-end and 3'-end of an RNA sequence encoding the target protein, respectively.

As used herein, the "attaching" does not only mean physically binding respective mRNA fragments but also includes a process of producing an mRNA sequence encoding a target protein, in which a 5'UTR of β-catenin and a 3'UTR of β-catenin or a fragment thereof is attached to the 5'-end and the 3'-end, respectively, through transcription from the DNA molecule.

In an embodiment of the present invention, the fragment of the 3'UTR of the present invention has a deletion of at least an intron 15 (In15) site in the full 3'UTR sequence.

In an embodiment of the present invention, the fragment of the 3'UTR of the present invention further has a deletion of an exon 16A (E16A) site.

In an embodiment of the present invention, the 3'UTR or a fragment thereof of the present invention may comprise an RNA sequence selected from the group consisting of the RNA sequence of SEQ ID NO: 6, the RNA sequence of SEQ ID NO: 7, the RNA sequence of SEQ ID NO: 9, the RNA sequence of SEQ ID NO: 10, the RNA sequence of SEQ ID NO: 11, the RNA sequence of SEQ ID NO: 12, the RNA sequence of SEQ ID NO: 13, the RNA sequence of SEQ ID NO: 14, the RNA sequence of SEQ ID NO: 15, and a combination thereof. More specifically, the 3'UTR or a fragment thereof of the present invention comprises an RNA sequence selected from the group consisting of the RNA sequence of SEQ ID NO: 9, the RNA sequence of SEQ ID NO: 11, the RNA sequence of SEQ ID NO: 13, and a combination thereof, and more specifically, may contains the RNA sequence of SEQ ID NO: 13.

In an embodiment of the present invention, the mRNA may be prepared through *in vitro* transcription.

As used herein, the term *"in vitro* transcription" refers to DNA-dependent RNA synthesis occurring outside a cell, typically in a test tube. Template DNA may be linearized with appropriate restriction enzymes prior to *in vitro* transcription. Examples of reagents used in the *in vitro* transcription of RNA typically comprise: polymerases such as bacteriophage-encoded RNA polymerases (T7, T3, SP6, or Syn5); ribonucleotide triphosphates (NTPs) for four bases (adenine, cytosine, guanine and uracil); optionally cap analogs; modified nucleotides; and RNase inhibitors, but are not limited thereto.

The present inventors identified that when mRNA comprising a 5'UTR of β-catenin and a 3'UTR of β-catenin or a fragment thereof of the present invention is synthesized through a DNA plasmid within a cell, as well as when using mRNA synthesized through *in vitro* transcription, the efficiency of the target protein can be improved, thereby allowing regulation of the intracellular expression pattern. Thus, it is proposed that mRNA comprising a 5'UTR of β-catenin and a 3'UTR of β-catenin or a fragment thereof of the present invention can be applied as a cancer vaccine mRNA structure that can regulatory immunity and be mass-produced.

Since the mRNA preparation method of the present invention employs the mRNA molecule with improved expression efficiency according to another aspect of the present invention, the overlap contents between the two inventions are applied in the same manner and the description thereof will be omitted to avoid the complexity of the specification.

The mRNA molecule capable of improving the expression efficiency of a target protein and promoting the movement of the target protein into the cytoplasm or cell membrane, the nucleic acid molecule encoding the mRNA molecule, the expression vector, the transformant, the host cell, and the composition of the present invention can be used to effectively enhance the expression of the target protein and promote the movement of the target protein into the cytoplasm or cell membrane, thereby ensuring the stable secretion of the target protein outside the cell.

### Advantageous Effects of Invention

Features and advantages of the present disclosure are summarized as follows.
i) The present invention provides an mRNA molecule comprising: (a) a coding region encoding a target protein; (b) a 5'untranslated region (UTR) of β-catenin attached to the 5'-end of the coding region; and (c) a 3'UTR of β-catenin or a fragment thereof attached to the 3'-end of the coding region.
ii) The present invention provides a nucleic acid molecule encoding the mRNA molecule.
iii) The present invention provides an expression construct comprising the nucleic acid molecule.
iv) The present invention provides a recombinant vector, comprising the expression construct.
v) The present invention provides an isolated host cell, comprising the recombinant vector.

The mRNA molecule that enhances the expression efficiency of a target protein and promotes the movement of the target protein into the cytoplasm or cell membrane, the nucleic acid molecule encoding the same, the expression vector, the transformant, the host cell, and the composition of the present invention can be used to effectively enhance the expression of the target protein. Furthermore, the present invention can promote the movement of the target protein into the cytoplasm or cell membrane, thereby ensuring the stable secretion of the target protein to the outside of a cell.

These advantages can be obtained not only through transformation and transfection using DNA plasmids but also for mRNA synthesized through *in vitro* transcription. As such, the use of the present invention can stably synthesize highly antigenic proteins and can present potential applications as an immunoregulatory cancer vaccine mRNA construct.

### Brief Description of Drawings

FIG. 1A is a schematic diagram of isomers of β-catenin 3'UTR.
FIG. 1B shows the results of RNA-FISH to analyze the intracellular β-catenin 3'UTR mRNA localization in the colon cancer cell lines HCT116, HT-29, and SW480 cells.
FIG. 1C shows the results of β-catenin 3'UTR isoform distribution in HCT116, HT-29, and SW480 colon cancer cell lines, quantified by nuclear (Nuc) and cytoplasmic (Cyt) localization.
FIG. 2A shows expression constructs comprising FLAG-tagged β-coding region (CR).
FIG. 2B shows the results of western blot analysis of expression efficiency after β-catenin was expressed in the stable SW480 cell line by using the constructs in FIG. 2A.
FIGS. 2C and 2D show the results of immunofluorescence analysis to observe the intracellular localization of expressed β-catenin after β-catenin was expressed in the stable SW480 cell line by using the constructs in FIG. 2A (FIG. 2C: cell population, and FIG. 2D: single cell).
FIG. 3A shows the results of analyzing the translation efficiency for each β-catenin UTR type in HEK293 cells by using β-catenin UTR luciferase reporter.
FIG. 3B shows the results of analyzing the translation efficiency for each β-catenin UTR type in SW480 cells by using β-catenin UTR luciferase reporter.
FIG. 4A shows expression constructs, comprising FLAG-tagged yellow fluorescent protein (YFP) (1: YFP, 2: 5'UTR + YFP, 3: 5'UTR + YFP + UTR-1, 4: 5'UTR + YFP + UTR-2, 5: 5'UTR + YFP + UTR-3, 6: 5 'UTR + YFP + ACTB 3'UTR).
FIG. 4B shows the results of western blot analysis of expression efficiency after YFP was expressed in U2OS cells by using the constructs in FIG. 4A (1: YFP, 2: 5'UTR + YFP, 3: 5'UTR + YFP + UTR-1, 4: 5'UTR + YFP + UTR-2, 5: 5'UTR + YFP + UTR-3, 6: 5'UTR + YFP + ACTB 3'UTR).
FIG. 4C shows the results of immunofluorescence analysis to observe intracellular localization of expressed YFP after YFP was expressed in U2OS cells by using the constructs in FIG. 4A.
FIG. 4D shows the results of quantification of the distribution of the nucleus (N) and cytoplasm (C) according to the intercellular localization of the YFP protein.
FIG. 4E shows the results of quantification of the distance from the nucleus to the cytoplasm where the YFP fluorescence was expressed.
FIG. 5 shows the results of analyzing expression efficiency promoting elements by using β-catenin 3'UTR fragment luciferase reporter in HEK293 cells.
FIG. 6A is a schematic diagram of FLAG YFP + 3'UTR fragment reporter expression constructs for analyzing an expression efficiency promoting and localizing element (1: YFP, 2: 5'UTR + YFP + F1, 3: 5'UTR + YFP + F3, 4: 5'UTR + YFP + F1+F3).
FIG. 6B shows the results of western blot analysis of protein expression after YFP was expressed in U2OS cells by using the fragment expression constructs in FIG. 6A (1: YFP, 2: 5'UTR + YFP + F1, 3: 5'UTR + YFP + F3, 4: 5'UTR + YFP + F1+F3).
FIG. 6C shows the results of analyzing the levels of proteins after YFP was expressed in U2OS cells by using the fragment expression constructs in FIG. 6A, the cell media were collected, and then the proteins were concentrated, followed by the release of proteins through extracellular vesicles (1: YFP, 2: 5'UTR + YFP + F1, 3: 5'UTR + YFP + F3, 4: 5'UTR + YFP + F1+F3)
FIG. 6D shows the results of cell fractionation of separating proteins in the cytoplasm and nucleus after YFP was expressed in U2OS cells by using the fragment expression constructs in FIG. 6A (1: YFP, 2: 5'UTR + YFP + F1, 3: 5'UTR + YFP + F3, 4: 5'UTR + YFP + F1+F3).
FIG. 7A shows the results of immunofluorescence analysis after the expression of YFP in U2OS cells using the fragment expression constructs in FIG. 6A.
FIG. 7B shows the results of quantification of the distance of YFP fluorescence expression (the distance of punta) from the nucleus to the cytoplasmic granule.
FIG. 8A is a schematic diagram of IVT-RNA-GFP constructs (1: GFP, 2: 5'UTR + GFP, 3: 5'UTR + GFP + UTR-1, 4: 5'UTR + GFP + UTR-2, 5: 5'UTR + GFP + ACTB 3'UTR).
FIG. 8B shows the results of RNA synthesis by loading IVT-mRNA onto a TBE agarose gel (1: GFP, 2: 5'UTR + GFP, 3: 5'UTR + GFP + UTR-1, 4: 5'UTR + GFP + UTR-2, 5: 5'UTR + GFP + ACTB 3'UTR).
FIG. 8C shows the results of western blotting of protein expression efficiency after IVT-RNA was transfected into U2OS cells (1: GFP, 2: 5'UTR + GFP, 3: 5'UTR + GFP + UTR-1, 4: 5'UTR + GFP + UTR-2, 5: 5'UTR + GFP + ACTB 3'UTR).
FIG. 8D shows the results of observation of GFP fluorescence through immunofluorescence analysis after IVT-RNA was transfected into U2OS cells (1: GFP, 2: 5'UTR + GFP, 3: 5'UTR + GFP + UTR-1, 4: 5'UTR + GFP + UTR-2).
FIG. 9A is a schematic diagram of an IVT-RNA-Luciferase construct.
FIG. 9B shows the results of confirming RNA synthesis by loading IVT-mRNA on TBE agarose gel.
FIG. 9C shows the results of the observation of luciferase and Rab7 through immunofluorescence analysis after IVT-RNA was transfected into HeLa cells.

### Best Mode for Carrying out the Invention

Hereinafter, the present invention will be described in more detail with reference to exemplary embodiments. These exemplary embodiments are provided only for the purpose of specifically illustrating the present invention, and therefore, according to the purpose of the present invention, it would be apparent to a person skilled in the art that these exemplary embodiments are not construed to limit the scope of the present invention.

### Examples

### Example 1: Analysis of expression efficiency and intracellular localization of β-catenin of each β-catenin UTR type

### Example 1-1. Analysis of intracellular localization for each 3'UTR isoform through RNA-fluorescence in situ hybridization (RNA-FISH)

### 1-1-1. Cell culture and transfection

HCT116 cells and SW480 cells (colon adenocarcinoma) were cultured in Roswell Park Memorial Institute (RPMI) 1640 medium (Hyclone). HEK293 cells (embryonic kidney), HT-29 cells, and U2OS cells were cultured in Dulbecco's Modified Eagle's medium (DMEM, Hyclone). Each medium contained 10% fetal bovine serum (FBS) and 1% antibiotics (Hyclone). Cell culture conditions were 5% CO₂ and 37°C. HEK293 cells were transfected using the PEI method (PEI MAX 40K, Polysciences, Inc.) according to the manufacturer's instructions, and the other cells were transfected using Lipofectamine 2000 (Invitrogen).

### 1-1-2. Fluorescence in situ hybridization (FISH)

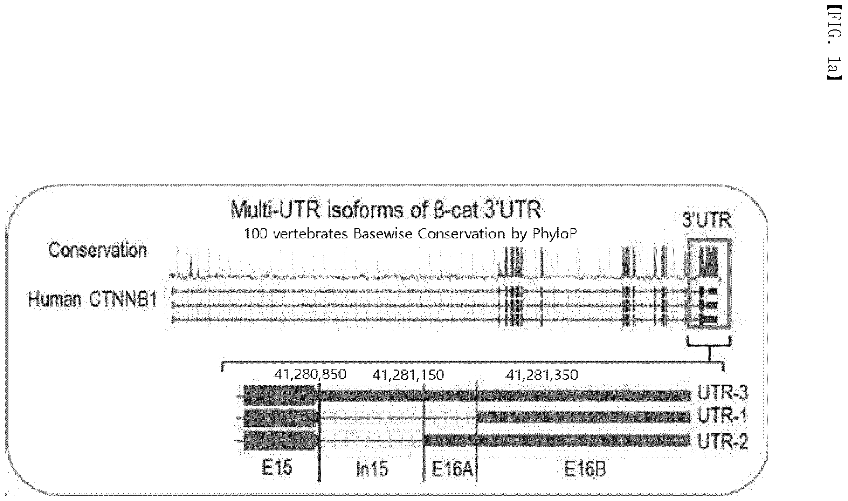

There are three alternative splicing isoforms of β-catenin 3'UTR (UTR-1, UTR-2, and UTR-3), all sharing the same coding region. UTR-3 is the longest isoform, comprising intron 15 (In15) and exon 16 (E16). UTR-2 is an intermediate-length isoform with intron 15 deleted. UTR-1 is the shortest isoform with intron 15 (In15) and exon 16A (E16A) deleted (FIG. 1A).

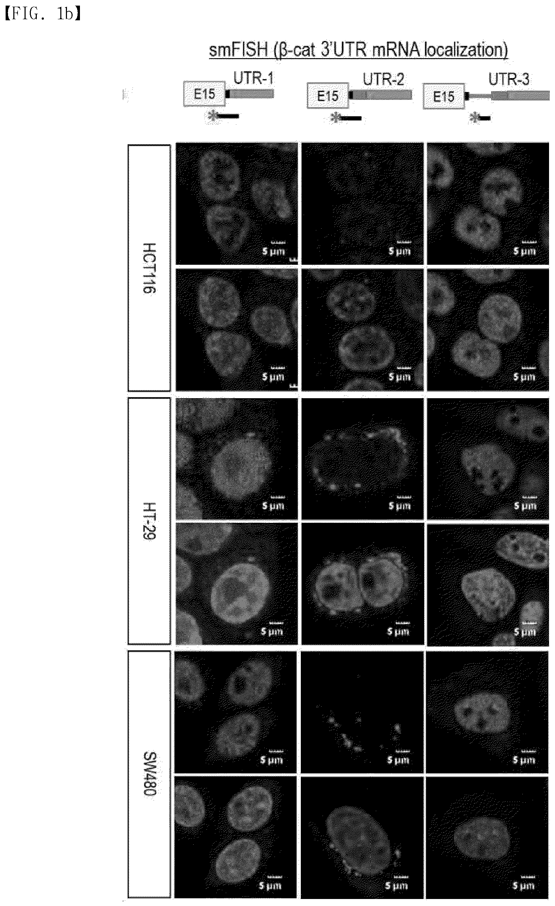

To investigate the effect of each different 3'UTR isoform on mRNA localization, fluorescent probes labeled with Cy3 or Cy5 for visualizing each β-catenin mRNA 3'UTR isoform were prepared (FIG. 1B and Table 1). RNA-Fluorescent In Situ Hybridization (RNA-FISH) was performed on the colon cancer cell lines HCT116, HT-29, and SW480 cells to analyze the intracellular 3'UTR mRNA localization.

For β-catenin mRNA-FISH experiments, the cells were cultured on coverslips. The cells were fixed with 4% paraformaldehyde at room temperature for 10 minutes, washed three times with 1× PBS, and permeabilized with 0.5 µg/ml digitonin. The samples were incubated with the prehybridization buffer containing 100 µg/ml Salmon sperm DNA (Stratagene) in 3% BSA in 4×SSC at 42°C for 20 minutes. The prehybridization buffer was removed, and the samples were incubated in a hybridization buffer (10% dextran sulfate in 4×SSC) containing the β-catenin 3'UTR mRNA probes (Table 1), prepared by the inventors at 42°C for 2 hours. Next, the samples were washed three times with 4×SSC, once with 2×SSC, once with 1×SSC, and once with 1× PBS, at room temperature, with each washing buffer for 5 minutes. All the samples were stained with DAPI by using VECTSHIELD mounting solution (Vector Laboratories) and mounted onto glass slides. The cells were visualized by confocal microscopy (Fluoview, FV3000; Olympus, Melville, NY).

**Table 1**

| **FISH probe sequence** | | | |
|---|---|---|---|
| **Probe** | **Detection** | **Sequence (5'>3')** | **SEQ ID NO** |
| 16B Junction | 3'UTR-1 | [5CY5]TGTTACTCCTAAAGGATG ATTTACAG[3'-dT] (26 mer) | 16 |
| 16A Junction | 3'UTR-2 | [5CY3]AGACAATACAGCTAAAGG ATGATTTACA[3'-dT] (28 mer) | 17 |
| Intron 15 | 3'UTR-3 | [5CY3]TAAACCACTCCCACCCTA CCAAC[3'-dT] (23 mer) | 18 |

As shown in FIG. 1B, in the three types of colon cancer cells, UTR-1 mRNA was primarily observed in the nucleus, though some localization in the cytoplasm was also detected. UTR-2 mRNA was mostly localized in the cytoplasm even though RNA appeared dispersed or granular. On the contrary, UTR-3 mRNA was observed to be localized only in the nucleus.

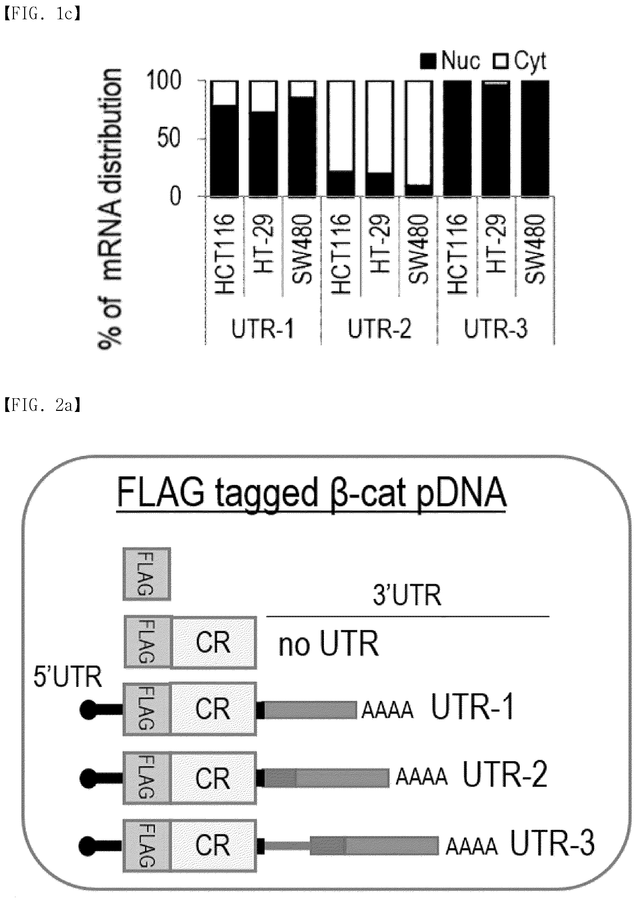

When the distribution according to the intracellular localization was quantified for the nucleus (Nuc) and cytoplasm (Cyt), about 80% or more of UTR-2 mRNA was present in the cytoplasm, as shown in the FISH image results for each UTR isomer (FIG. 1C).

It can be therefore identified that the intercellular mRNA localization was different for each 3'UTR isoform.

### Example 1-2. Analysis of expression efficiency and intracellular protein localization for each β-catenin UTR type through FLAG β-catenin coding region + UTR reporter

### 1-2-1. Analysis of expression efficiency for each β-catenin UTR type through FLAG β-catenin coding region + UTR reporter

To confirm whether the co-expression of 5'UTR and 3'UTR isoforms had an effect of improving the expression efficiency in an actual cell environment, expression constructs containing FLAG-tagged β-catenin coding region (CR) were constructed (FIG. 2A).

The pCAN Myc-tagged β-catenin coding region (CR) plasmid was provided from Dr. Paul McCrea (University of Texas, Austin). For β-catenin expression, DNA was amplified from the provided pCAN Myc β-catenin CR (Coding Region) clone by using β-cat CR F and β-cat CR R primers. The PCR product was digested with BamHI and XbaI enzymes and cloned into the p3xFLAG-CMV-10 vector. PCR amplification was performed from the pGL3/Luc β-catenin 5'UTR clone by using a β-cat 5'UTR primer set, and the PCR product was digested with BamHI and BglII, and then cloned upstream of 3xFLAG-β-cat CR clone. β-cat CR, including 5'UTR, was amplified by PCR, digested with BamHI and XbaI enzymes, and cloned into the pCS2 vector. Three β-cat 3'UTRs were amplified from three previously constructed pGL3/Luc β-catenin 3'UTR clones by using β-cat CR + 3'UTR primer sets, digested with XbaI and ApaI enzymes, and cloned into the pCS2 β-cat 5'UTR 3xFLAG β-cat CR clone constructed above. The used primers are shown in Table 2.

PCR amplification was performed from the clones constructed above by using primers designed for retroviral applications (Table 2), and the PCR products were digested with BamHI and SalI enzymes, transferred into the pBABE vector by cloning, and transfected into SW480 colon cancer cells, thereby establishing a cell line with stable and continuous expression of β-catenin.

**Table 2**

| **Primer** | **Restriction enzyme** | **Sequence (5'→3')** | **SEQ ID NO** |
|---|---|---|---|
| β-catenin Coding Region (CR) Forward primer | BamHI | GCGGGATCCAATGGCTACT CAAGCTGATTTG | 19 |
| β-cat Coding Region (CR) Reverse primer | | TGGGATCCAGGTCAGTATC AAACCAGGC | 20 |
| β-cat 5'UTR Forward primer | BamHI | GGGATCCAGGATACAGCGG CTTCTGCG | 21 |
| β-cat 5'UTR Reverse primer | BglII | ATGAGATCTTGTCCACGCT GGATTTTCAAAACA | 22 |
| β-cat CR+3'UTR-1 Forward primer | XbaI | GCTCTAGAATCATCCTTTA GGAGTAACAATAC | 23 |
| β-cat CR+3'UTR-2 Forward primer | | GCTCTAGAATCATCCTTTA GCTGTATTGTCT | 24 |
| β-cat CR+3'UTR-3 Forward primer | | GCTCTAGAATCATCCTTTA GGTAAGAAGTTTA | 25 |
| β-cat CR+3'UTR Reverse primer | ApaI | ATGGGCCCCAATCGAATGA ATTAAAAGTTT | 26 |
| Retro viral β-cat 5'UTR Forward primer | BamHI | CCGGATCCAGGATACAGCG GCTTCTGC | 27 |
| Retro viral β-cat 3'UTR Reverse primer | SalI | CAGGGTCGACCAATCGAAT GAATTAAAAGTTTAATTCT | 28 |

The cell line was analyzed for protein expression efficiency by western blotting. Each type of stably expressed cell was harvested by washing with 1× PBS, followed by centrifugation to pellet the cells, and 1X passive lysis buffer (Promega) was then added to lyse the cells. The lysed cells were centrifuged at 13,000 rpm for 15 minutes to separate the cell debris, and only the supernatant lysate was transferred to a new tube. The extracted protein lysate was suspended in 4X sample buffer (200 mM Tris-HCl, pH 6.8, 8% SDS, 40% glycerol, 0.4% bromophenol blue, 400 mM DTT). The protein sample was separated on 8% and 12% SDS-PAGE gels and transferred to the polyvinylidene difluoride membrane (PVDF) (Millipore). The membrane was blocked with 5% skim milk for 1 hour, and then incubated overnight at 4°C with primary antibodies. The antibodies used were as follows: anti-FLAG M2 (F3165, Sigma-Aldrich) and anti-β-actin (ab6276, Abcam).

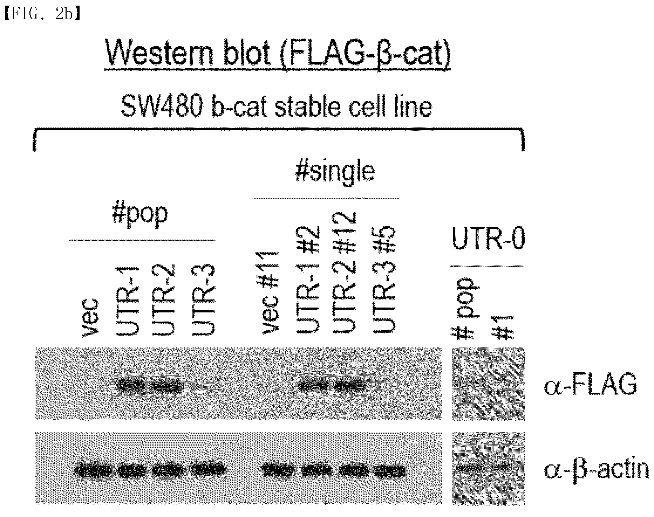

As a result, as shown in FIG. 2B, the expression efficiency was higher for the presence of both 5'UTR and UTR-1 or both 5'UTR and UTR-2 compared with UTR-0 (the presence of only the coding region). The presence of both 5'UTR and UTR-3 showed different expression levels depending on the type of clone, with low expression efficiency.

### 1-2-2. Analysis of intracellular protein localization for each β-catenin UTR type through FLAG β-catenin coding region + UTR reporter

To observe the intracellular localization of the expressed β-catenin protein, immunofluorescence analysis was conducted by staining FLAG antibody with FITC secondary antibody.

For β-catenin protein immunofluorescence experiments, the cell lines were cultured on coverslips, and each stably expressed cell type was fixed with 4% paraformaldehyde at room temperature for 10 min, washed three times with 1× PBS, and permeabilized with 100% methanol. The samples were treated with 50 mM glycine to suppress formaldehyde, washed with 1× PBS, and blocked with 5% BSA at room temperature for 30 minutes. The primary antibody was diluted 1:1000 in 5% BSA and added to the sample, followed by incubation for 16 hours. Then, the secondary antibody was diluted 1:1000 in 5% BSA and added to the sample, followed by incubation for 2 hours. The samples were washed three times with 1× PBS, stained with DAPI by using VECTSHIELD mounting solution (Vector Laboratories), and mounted onto glass slides. The cells were visualized by confocal microscopy (Fluoview, FV3000; Olympus, Melcille, NY).

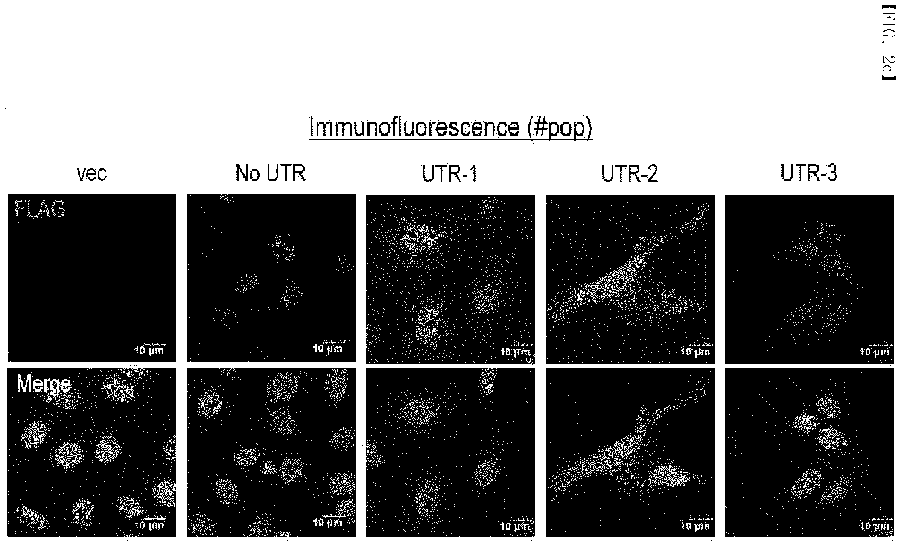

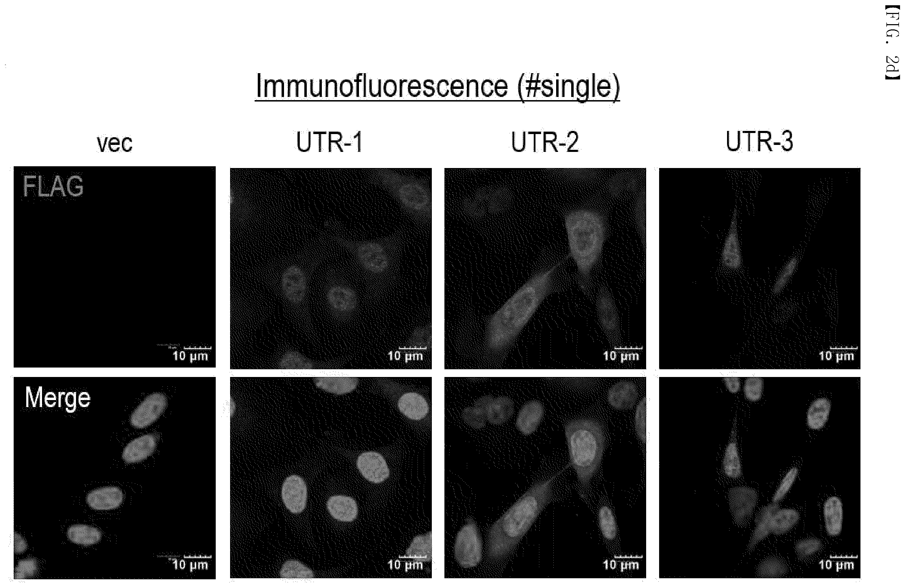

The results are shown in FIGS. 2C and 2D. In SW480 cells, for UTR-0, the expression level was low and the protein was mostly observed in only the nucleus. However, for UTR-1 and UTR-2, the expression level was high and the protein was observed in the cytoplasm as well as the nucleus. Especially, for UTR-2, the cell morphology was elongated and the expression to the end of a cell was clearly observed. For UTR-3, there was a difference in expression depending on the clone, with low expression efficiency, similar to the results in FIG. 1B (FIGS. 2C and 2D). It can be therefore identified that the type of β-catenin 3'UTR mRNA isoform plays an important role in the expression efficiency and intracellular protein localization.

### Example 2: Analysis of protein expression efficiency for each β-catenin UTR type through β-catenin UTR luciferase reporter

The different intracellular localization of mRNA expression for each of β-catenin 3'UTR isoforms, as observed in Example 1, suggest that protein expression may be regulated by 3'UTR. To verify the possibility, a luciferase reporter was constructed to assess the translation efficiency of the β-catenin 3'UTR.

To construct the β-catenin UTR luciferase reporter, 5'UTR and three 3'UTRs (UTR-1, UTR-2, UTR-3) were amplified from β-catenin mRNA of SW480 cells by RT-PCR using primers for luciferase assay listed in Table 3 below. The 5 'UTR PCR product was digested with HindIII and NcoI enzymes and cloned into the upstream of the luciferase gene in the pGL3/Luc vector. Each of the three 3'UTR PCR products was digested with XbaI enzyme and cloned into the downstream of the luciferase gene in the pGL3/Luc vector. To construct β-catenin luciferase reporters comprising 5'UTR and each of three isoforms of 3'UTR, the amplification was performed from the three 3'UTR clones constructed above by the In-Fusion HD Cloning PCR method using In-Fusion primers. The PCR products were digested with XbaI enzyme and cloned into the downstream of the luciferase gene of the pGL3/Luc β-catenin 5 'UTR clone.

**Table 3**

| **Primer** | **Restriction enzyme** | **Sequence (5'→3')** | **SEQ ID NO** |
|---|---|---|---|
| β-catenin 5'UTR Forward primer | HindIII | GGAAGCTTAGGATACAGCGGCT TCTGC | 29 |
| β-cat 5'UTR Reverse primer | NcoI | GGCCATGGTGTCCACGCTGGAT TTTCA | 30 |
| β-cat 3'UTR Forward primer | XbaI | ATTCTAGAGATACTGACCTGTA AATCATCC | 31 |
| β-cat 3'UTR Reverse primer | | CATCTAGAAATGAATTAAAAGT TTAATTCTGAACC | 32 |
| In-Fusion β-cat 3'UTR-1 Forward primer | | GCCGTGTAATTCTAGAATCATC CTTTAGGAGTAACAA | 33 |
| In-Fusion β-cat 3'UTR-2 Forward primer | | GCCGTGTAATTCTAGAATCATC CTTTAGCTGTATTGT | 34 |
| In-Fusion β-cat 3'UTR-3 Forward primer | | GCCGTGTAATTCTAGAATCATC CTTTAGGTAAGAAGT | 35 |
| In-Fusion β-cat 3'UTR Reverse primer | | CCGCCCCGACTCTAGACAATCG AATGAATTAAAAGT | 36 |

For the luciferase assay to measure the translation efficiency of β-catenin mRNA UTR, HEK293 cells were cultured in 12-well plates. The respective pGL3/Luc β-catenin UTR reporters were co-transfected along with the pRL-TK vector into the cells. The transfected cells were harvested by washing them with 1× PBS, followed by cell down through centrifugation, and then 1X passive lysis buffer (Promega) was added to lyse the cells. The lysed cells were centrifuged at 13,000 rpm for 15 minutes to achieve cell down, and only the lysate in the supernatant was transferred to a new tube. Each sample was then analyzed by a GLOMAX20/20 luminometer using the Dual-Luciferase Assay Kit (Promega).

SW480 cells were also subjected to luciferase assay for measuring the translation efficiency of β-catenin mRNA UTR by the same method as in the HEK293 cells.

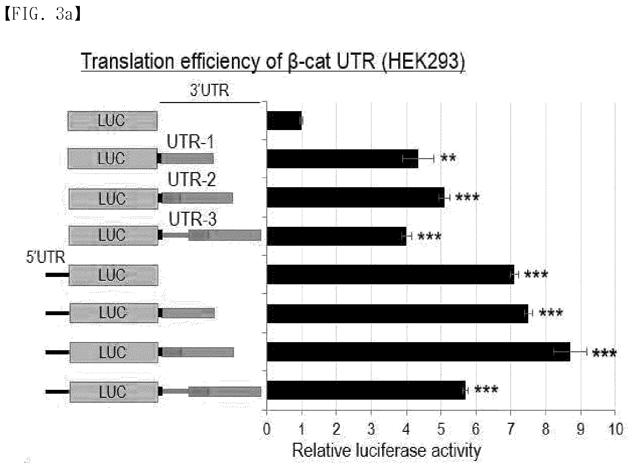

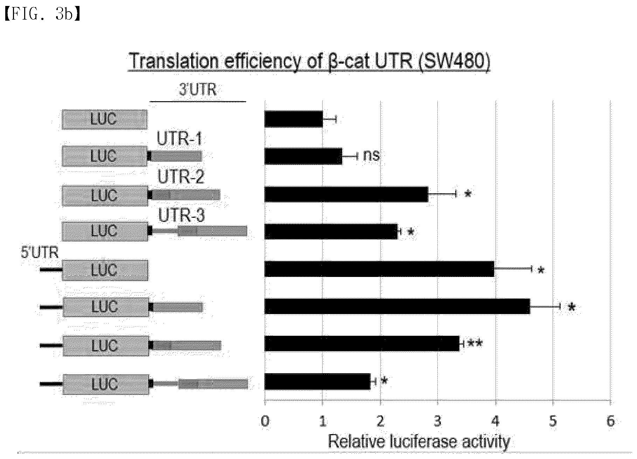

The results are shown in FIGS. 3A and 3B. As shown in FIGS. 3A and 3B, in both HEK293 cells and SW480 cells, for the presence of only 3'UTR, the translation efficiency for each isoform type was generally low, with no significant difference between the isoforms. However, when both the 5'UTR and 3'UTR were present, similar to actual cell situations, a significant increase in translation efficiency was observed. Particularly, the translation efficiency was further increased when both the 5'UTR and UTR-1 were present, and when both the 5'UTR and UTR-2 were present, whereas the presence of UTR-3 led to only minor increase in translation efficiency (FIGS. 3A and 3B). It can be therefore identified that UTR-1 and UTR-2 mRNAs are abundantly localized in the cytoplasm and thus play an important role in enhancing translation efficiency.

### Example 3: Analysis of translation efficiency for each β-catenin UTR type through FLAG yellow fluorescent protein (YFP) + 3'UTR overexpressed reporter

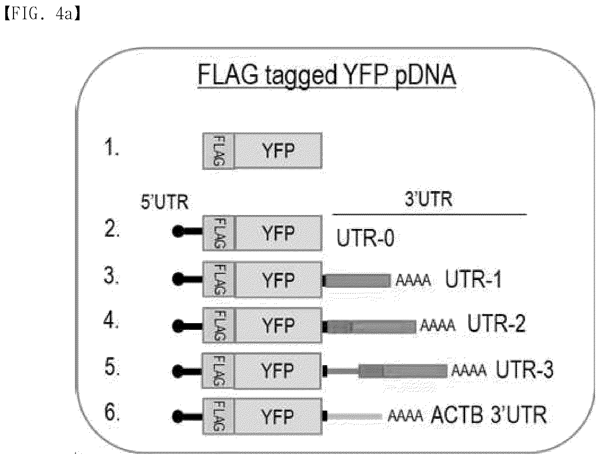

To more accurately determine the function of β-catenin 3'UTR mRNA itself, expression constructs were constructed in which yellow fluorescent protein (YFP) replaced the β-catenin coding region and was tagged with FLAG (FIG. 4A).

First, an expression construct comprising the β-catenin coding region (CR) tagged with FLAG was constructed and the coding region was replaced with yellow fluorescent protein (YFP).

The pCAN Myc-tagged β-catenin coding region (CR) plasmid was provided byDr. Paul McCrea (University of Texas, Austin). For β-catenin overexpression, DNA was amplified from the provided pCAN Myc β-catenin CR clone by using β-cat CR F and β-cat CR R primers. The PCR product was digested with the BamHI enzyme and cloned into the p3xFLAG-CMV-10 vector. The 3xFLAG-β-cat CR, comprising 5'UTR upstream thereof, was amplified by PCR and cloned into the pCS2 vector. Three β-cat 3'UTRs were amplified from three previously constructed pGL3/Luc β-catenin 3'UTR clones by using β-cat CR + 3'UTR primer sets, then digested with XbaI and ApaI enzymes, and cloned into the pCS2 β-cat 5'UTR 3xFLAG β-cat CR clone constructed above. The used primers are shown in Table 2 in Example 1-2.

Thereafter, the existing EcoRI enzyme site was replaced with the MluI enzyme site by site-directed mutagenesis, and DNA was amplified from the pEBB YFP clone and cloned into the pCS2 clones after digestion with MluI and XbaI enzymes. Additionally, for comparison of the expression efficiency of β-cat UTR, the 3'UTR gene of β-actin (ACTB 3'UTR, SEQ ID NO: 37), one of the housekeeping genes that are well expressed in any situation, was cloned into the pCS2 β-cat 5'UTR 3xFLAG YFP clone by the same method. The used primers are shown in Table 4.

**Table 4**

| **Primer** | **Restricti on enzyme** | **Sequence (5'→3')** | **SEQ ID NO** |
|---|---|---|---|
| MluI mutation Forward primer | MluI | GCCGCGACGCGTTCGATAGA TCCA | 38 |
| MluI mutation Reverse primer | MluI | TGGATCTATCGAACGCGTCG CGGC | 39 |
| YFP Forward primer | MluI | GACGCGTATGGTGAGCAAGG GCGA | 40 |
| YFP Reverse primer | XbaI | TCTAGATTAAGCTCGAGATC TGAGTCCGG | 41 |
| YFP β-actin 3'UTR Forward primer | XbaI | CCCTCTAGATAGGCGGACTA TGACTTAGTTGCGT | 42 |
| YFP β-actin 3'UTR Reverse primer | ApaI | GCGGGGCCCTCATTTTTAAG GTGTGCACTTTTA | 43 |

The YFP constructs were transfected into U2OS cells, and protein expression efficiency was analyzed by Western blot.

The transfected cells were harvested by washing with 1× PBS, followed by cell down through centrifugation, and then 1X passive lysis buffer (Promega) was added to lyse the cells. The lysed cells were centrifuged at 13,000 rpm for 15 minutes to achieve cell down, and only the lysate in the supernatant was transferred to a new tube. The extracted protein lysate was suspended in 4X sample buffer (200 mM Tris-HCl, pH 6.8, 8% SDS, 40% glycerol, 0.4% bromophenol blue, 400 mM DTT). The protein sample was subjected to separation on 8% and 12% SDS-PAGE gels and transferred to the polyvinylidene difluoride membrane (PVDF) (Millipore). The membrane was blocked with 5% skim milk for 1 hour, and then incubated overnight at 4°C with primary antibodies. The used antibodies are as follows:

anti-FLAG M2 (F3165, Sigma-Aldrich), anti-GFP (SC-126, Santacruz), and anti-β-actin (ab6276, Abcam) (Table 5).

**Table 5**

| **Manufacturer** | **Antibody** |
|---|---|
| Sigma-Aldrich F3165 | FLAG M2 |
| Santacruz SC-126 | GFP |
| Abcam ab6276-100 | Beta-actin |

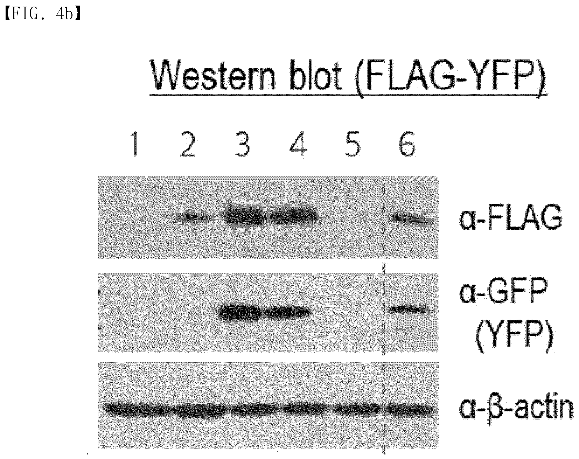

As a result, when both the 5'UTR and UTR-1 were present, or both the 5'UTR and UTR-2 were present, the expression efficiency was high, showing even higher efficiency than the 3'UTR of β-actin (FIG. 4B).

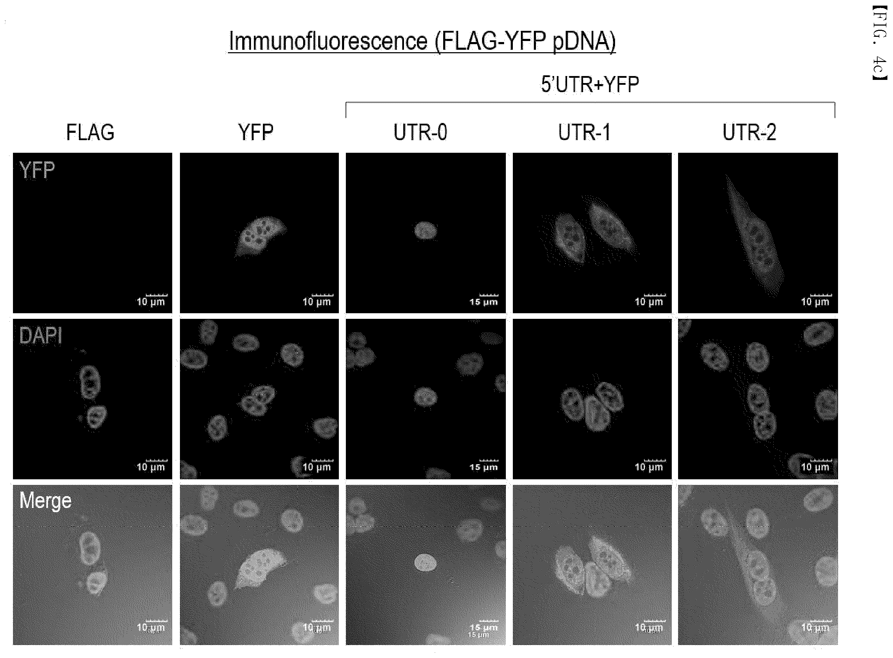

The YFP constructs were transfected into osteosarcoma U2OS cells, and immunofluorescence analysis was performed to observe YFP fluorescence. The results are shown in FIG. 4C. For the presence of only YFP coding region, expression was observed in both the cytoplasm and nucleus, but the expression in the nucleus was slightly higher than the expression in the cytoplasm. The proportion of YFP observed in the nucleus was higher for the presence of 5'UTR + YFP (UTR-0) than the presence of only the YFP coding region. The expression was observed in both the nucleus and the cytoplasm for 5'UTR + YFP + UTR-1 and 5'UTR + YFP + UTR-2, similar to the presence of only the YFP coding region. Especially, for 5'UTR + YFP + UTR-2, the YFP was expressed in the overall cytoplasm from the nucleus (FIG. 4C).

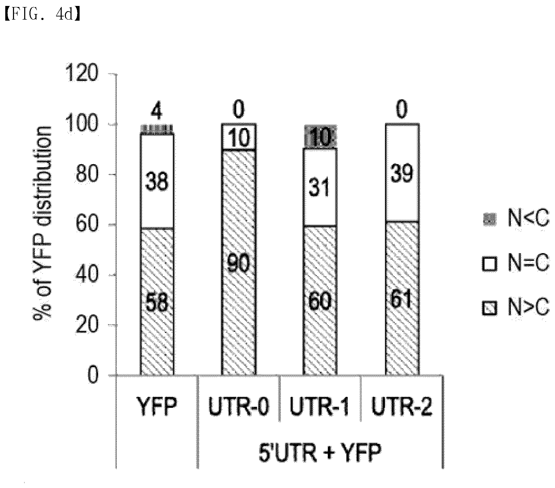

The distribution of YFP protein in the nucleus (N) and cytoplasm (C) was quantified based on its cellular localization, and the results are shown in FIG. 4D. For all the cases except for 5'UTR + YFP (UTR-0), the YFP protein was distributed between the nucleus and cytoplasm at an approximate ratio of 6:4 (FIG. 4D).

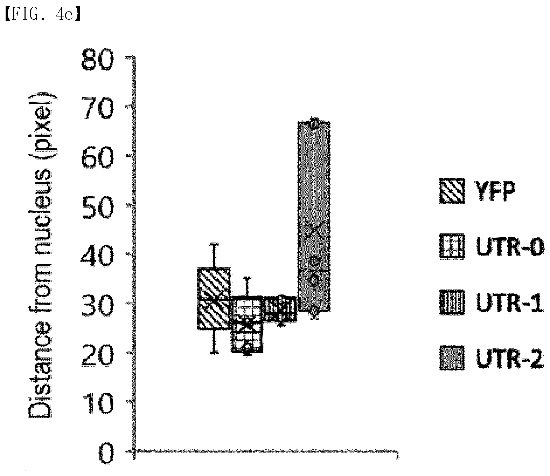

The distance of YFP fluorescence expression from the nucleus to the cytoplasm was quantified, and the results are shown in FIG. 4E. No difference was observed among the β-catenin 3'UTR isoforms when only the distribution was quantified as shown in FIG. 2D, but as a result of quantifying the distance to the cytoplasm as shown in FIG. 4E, YFP was expressed to the cell periphery for 5'UTR + YFP + UTR-2 as shown in the immunofluorescence analysis image, indicating that the distance to the cytoplasm, where the protein was expressed, was the longest (FIG. 4E).

Through this, It was confirmed that the expression efficiency of the β-catenin 3'UTR is excellent, and especially, that UTR-2 mRNA has a significant effect on cell morphology and the expression localization.

### Example 4: Analysis of expression efficiency promotion element by using β-catenin 3'UTR fragments

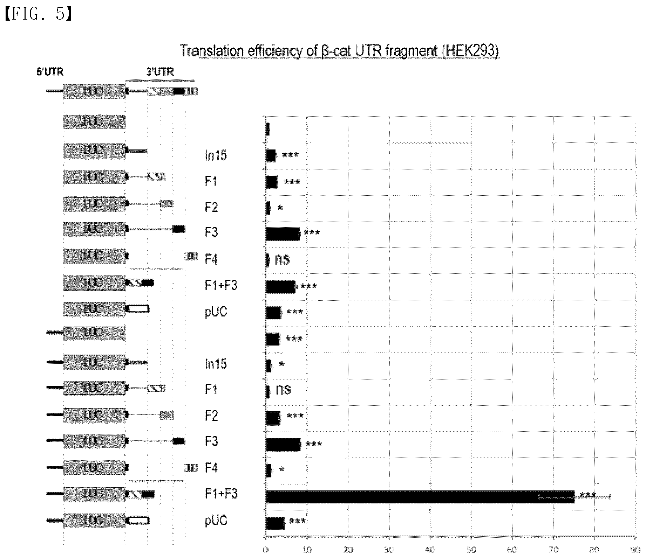

To identify which site of the β-catenin 3'UTR mRNA plays the most significant role in enhancing the expression efficiency, UTR-1 and UTR-2 were fragmentated, and luciferase reporter expression constructs were generated (FIG. 5).

F1 (fragment 1, SEQ ID NO: 9, the entire E16A site), F2 (fragment 2, SEQ ID NO: 10, a portion of E16B site), F3 (fragment 3, SEQ ID NO: 11, a portion of E16B site), F4 (fragment 4, SEQ ID NO: 12, a portion of E16B site), and F1+F3 (fragment 1 + fragment 3, SEQ ID NO: 13) were generated as fragments.

To compare expression efficiency, an expression construct with the Intron 15 (In15) region (SEQ ID NO: 3) inserted thereinto was generated, and a construct with pUC (SEQ ID NO: 44) inserted thereinto was constructed as a control (FIG. 5).

As for F1, F2, F3, F4, and F1+F3, DNAs were amplified using corresponding F and R primers, digested with XbaI and SalI enzymes, and inserted into the previously generated pGL3/Luc clone and pGL3/Luc β-catenin 5'UTR clone. For DNA amplification, pCS2 β-cat 5'UTR 3xFLAG β-cat CR + 3'UTR-3 clone was used as a template. The used primers are shown in Table 6 below.

**Table 6**

| **Primer** | **Restriction enzyme** | **Sequence (5'→3')** | **SEQ ID NO** |
|---|---|---|---|
| Luciferase β-catenin 3'UTR-2 F1 Forward primer | XbaI | CCCTCTAGACTGTATTGTCT GAACTTGCATT | 45 |
| Luciferase β-catenin 3'UTR-2 F1 Reverse primer | SalI | CCCGTCGACGAGAGACTTAA AAAACAGTTACTGC | 46 |
| Luciferase β-catenin 3'UTR-1 F2 Forward primer | XbaI | CCCTCTAGAGAGTAACAATA CAAATGGATTTTG | 47 |
| Luciferase β-catenin 3'UTR-1 F2 Reverse primer | SalI | CCCGTCGACTGTTCTACACC ATTACTCAATTCT | 48 |
| Luciferase β-catenin 3'UTR-1 F3 Forward primer | XbaI | CCCTCTAGAGTAACTGTTTT TTAAGTCTCTCGT | 49 |
| Luciferase β-catenin 3'UTR-1 F3 Reverse primer | SalI | CCCGTCGACACACCTCTTAC TGATTTACCCTA | 50 |
| Luciferase β-catenin 3'UTR-1 F4 Forward primer | XbaI | CCCTCTAGATGGTCCAATTA GTTTCCTTTTTAA | 51 |
| Luciferase β-catenin 3'UTR-1 F4 Reverse primer | SalI | CCCGTCGACCAATCGAATGA ATTAAAGTTTA | 52 |
| Luciferase β-catenin In15 Forward primer | XbaI | CCCTCTAGATCTAGAGATAC TGACCTGTAAATC | 53 |
| Luciferase β-catenin In15 R | SalI | CCCGTCGACCTGTATAAAGA GAGAAAGGCTG | 54 |
| Luciferase pUC Forward primer | XbaI | GGTCTAGAATACCTGTCCGC CTTTCTCC | 55 |
| Luciferase pUC Reverse primer | SalI | CCGTCGACCGCTCTGCTAAT CCTGTTA | 56 |

The results of protein expression analysis following transfection of the above fragment expression constructs into HEK293 cells are shown in FIG. 5. As shown in FIG. 5, the translation efficiency was generally low when 3'UTR fragments were present, similar to the control group with pUC inserted. However, in the presence of the F3 fragment of the 3'UTR (F3 and F1+F3), a higher expression deficiency was observed. Additionally, when the 5'UTR was present, expression efficiency increased compared to when only the 3'UTR fragments were present. Particularly, when the 5'UTR was present together with F2, F3, or F1+F3 of 3'UTR, expression efficiency was elevated, and notably, expression efficiency was very high when both the 5'UTR and F1+F3 were present. It can be therefore identified that F3 among the 3'UTR fragments is the most important element for enhancing expression efficiency.

### Example 5: Analysis of element for expression efficiency promotion and expression localization through FLAG yellow fluorescent protein (YFP) + 3'UTR fragment reporters

In Example 3 described above, it was confirmed that in the case of 5'UTR + YFP + UTR-2, the protein was expressed up to the cell periphery, indicating that UTR-2 is the most influential element for cell morphology and expression localization. In Example 2, it was also confirmed that UTR-2 plays a significant role in expression efficiency. The present inventors predicted that UTR-2 is the only one comprising E16A among the 3'UTR isoforms and thus F1, a fragment corresponding to E16A among the 3'UTR fragments, would have a significant effect on the cell morphology and expression localization, like UTR-2.

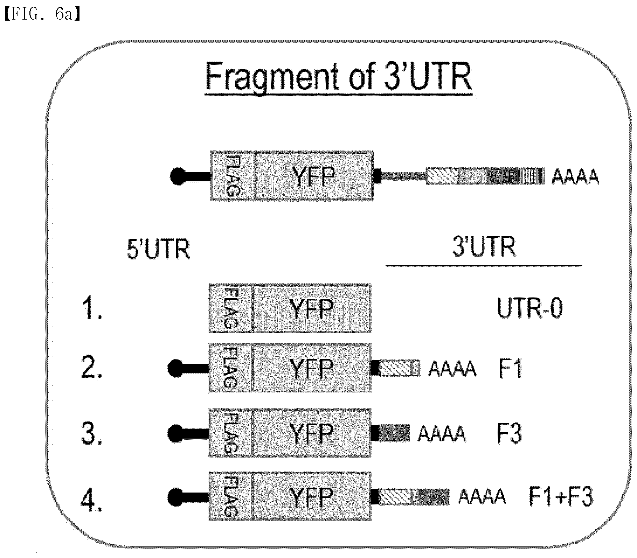

Using F1 along with F3, which showed a high expression efficiency in Example 5, YFP constructs were generated (FIG. 6A). DNA was amplified using the corresponding Forward and Reverse primers, digested with XbaI and ApaI enzymes, and cloned inserted into the pCS2 β-cat 5'UTR 3xFLAG YFP clone in Example 3. For DNA amplification, the pCS2 β-cat 5'UTR 3xFLAG β-cat CR+ 3'UTR-3 clone was used as a template. The used primers are shown in Table 7 below.

**Table 7**

| **Primer** | **Restriction enzyme** | **Sequence (5'→3')** | **SEQ ID NO** |
|---|---|---|---|
| YFP 3'UTR-2 F1 Forward primer | XbaI | ATATCTAGACTGTATTGTC TGAACTTGCATT | 57 |
| YFP 3'UTR-2 F1 Reverse primer | ApaI | ATAGGGCCCGAGAGACTTA AAAAACAGTTACTGC | 58 |
| YFP 3'UTR-1 F3 Forward primer | XbaI | CCCTCTAGAGTAACTGTTT TTTAAGTCTCT [?] CGT | 59 |
| YFP 3'UTR-1 F3 Reverse primer | ApaI | CGCGGGCCCTAGGGTAAAT CAGTAAGAGGTGT | 60 |
| YFP 3'UTR-1 F1+F3 Forward primer | ApaI | ATAGGGCCCGTAACTGTTT TTTAAGTCTCTCGT | 61 |
| YFP 3'UTR-1 F1+F3 Reverse primer | KpnI | ATAGGTACCTAGGGTAAAT CAGTAAGAGGTGT | 62 |
| ApaI deletion Forward primer | • | GTCCTTTTTGGTCGAGGTA ACTGTTTTTTAAG | 63 |
| ApaI deletion Reverse primer | • | CTTAAAAAACAGTTACCTC GACCAAAAAGGAC | 64 |

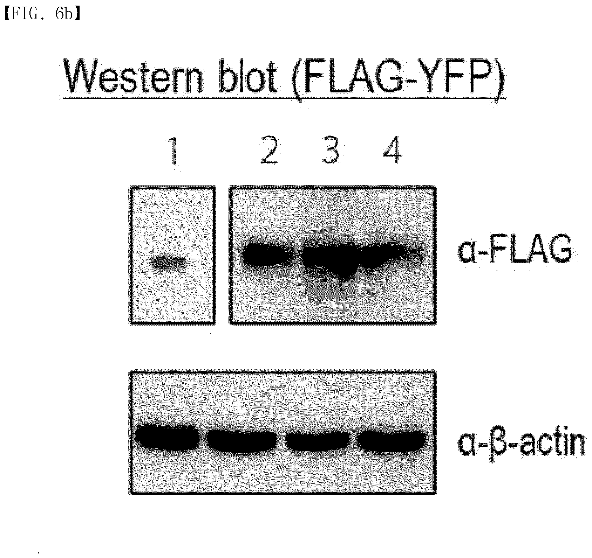

After transfecting osteosarcoma U2OS cells with the fragment expression constructs and analyzing protein expression by western blot, it was confirmed that F3 showed the highest expression efficiency among UTR-1 fragments (FIG. 6B).

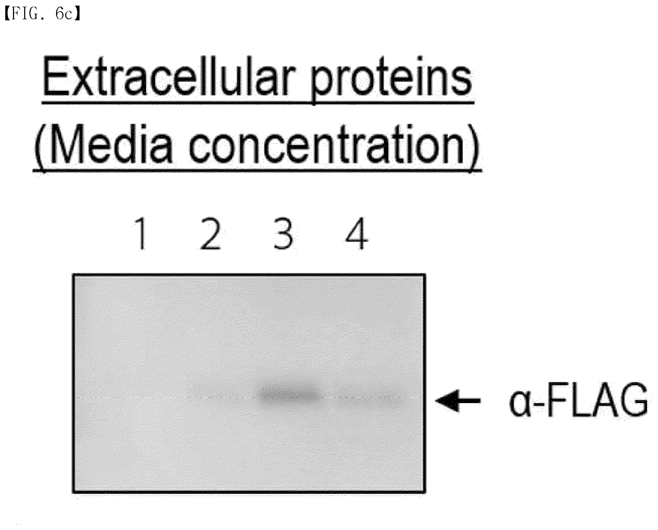

To analyze the amount of protein secreted through extracellular vesicles after expression, U2OS cells were transfected with the fragment expression constructs, and the cell media wes collected and the protein was concentrated through a Centricon filter. The results are shown in FIG. 6C. F3, which showed a high level of protein expression, also secreted a large amount of protein outside the cell, and F1+F3 showed the similar results although a smaller amount was secreted compared with F3 (FIG. 6C).

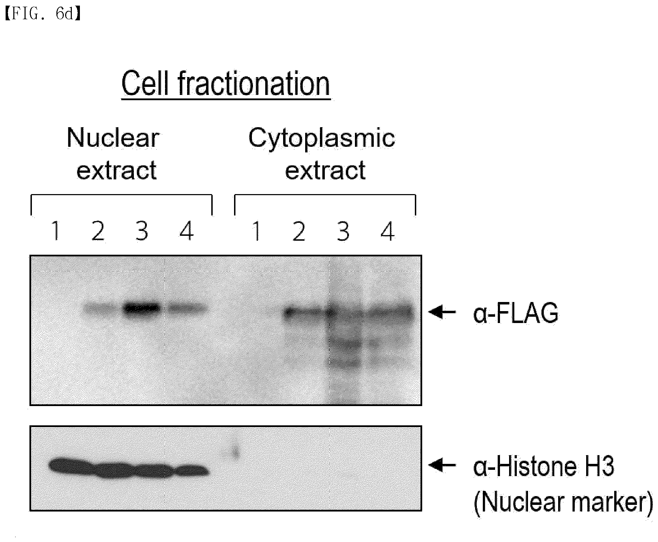

To analyze the intercellular localization of expression of proteins, U2OS cells were transfected with the fragment expression constructs, and followed by a cell fractionation method of separating the proteins in the cytoplasm and nucleus. The results are shown in FIG. 6D. In the absence of UTR (1: YEP), expression was much lower than with the UTR fragments, and the expression of F3 was high as in the results shown in FIG. 6B. However, many bottom bands of F3 (3: 5'UTR + YFP + F3) were detected in the cytoplasmic extract, indicating that F3 showed a high proteolytic activity. However, fewer bottom bands of F1 (2: 5'UTR + YFP + F1) were detected compared with F3 in the cytoplasmic extract, indicating that F1 showed a low proteolytic activity. F1+F3 (4: 5'UTR + YFP + F1 + F3) showed a lower proteolytic activity compared with the presence of only F3 (FIG. 6D).

As such, the present inventors established that the protein expression efficiency of F3 was high and the proteolytic activity of F1 was low in the cytoplasm, suggesting that the F1+F3 protein resulting from a combination of F1 and F3 described above would be stably expressed in the cytoplasm.

### Example 6: Analysis of expression localization element through FLAG yellow fluorescent protein (YFP) + 3'UTR fragment reporters

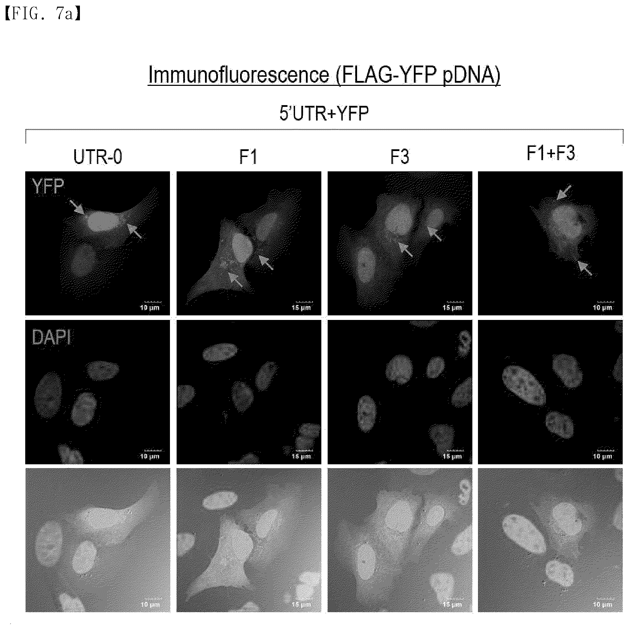

Based on the analysis results in Example 6, the fragment expression constructs were transfected into U2OS cells, followed by immunofluorescence analysis to observe YFP fluorescence. The results are shown in FIG. 7A. As shown in FIG. 7A, all the constructs were highly expressed in the nucleus but were observed to show different patterns in the cytoplasm. Granules were observed around the nucleus, for F3 and the absence of UTR (UTR-0). However, in the case of F1, expression was observed in the cytoplasm farther from the nucleus and extended to the outer membrane of the cell, and a similar pattern was observed with the combination of F1+F3.

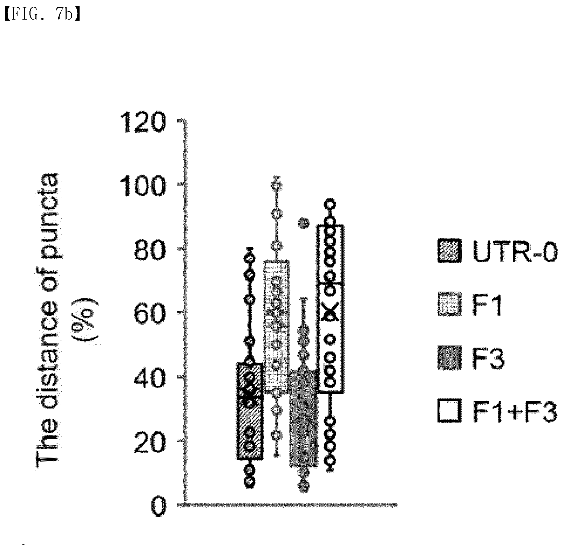

The distance of YFP fluorescence expression from the nucleus to the cytoplasmic granules was quantified, it was confirmed that the expressed protein was located farthest from the nucleus in the presence of F1 or F1+F3 (FIG. 7B).

As such, the present inventors have established and validated though the above-described examples that F1+F3 promotes the movement of a target protein to the cytoplasm or cell membrane, consequently enabling the target protein to be stably secreted to the extracellular region.

### Example 7: Analysis of expression efficiency and expression localization for each UTR type of in vitro transcribed RNA

### Example 7-1. Analysis of expression efficiency and localization for each in vitro-RNA β-catenin 3'UTR type by using green fluorescent protein (GFP)

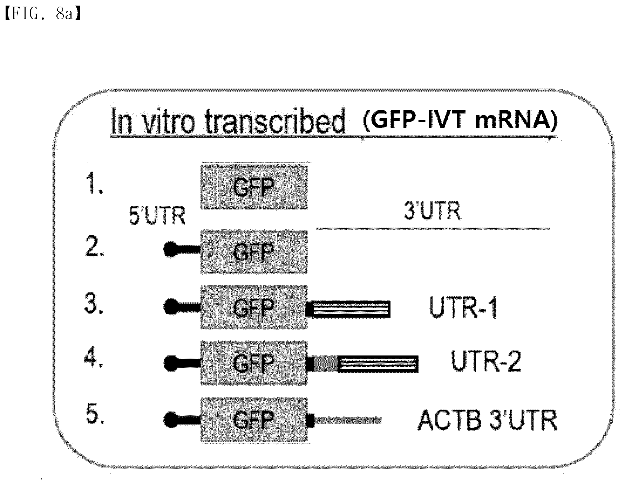

To analyze whether β-catenin 3'UTR had an effect on the intracellular expression pattern of *in vitro-RNA* (IVT-RNA), similar to the results confirmed using the DNA plasmid, IVT-RNA-GFP constructs were constructed (FIG. 8A).

The EGFP coding sequence was inserted into GEM-11zf(+), a clone containing a T7 promoter that enables the operation of bacterial T7 RNA polymerase, after digestion with SalI and BamHI enzymes. To investigate the functions of 5'UTR and 3'UTR of CTNNB1 in the production of enhanced green fluorescent protein (EGFP), 5'UTR of CTNNB1 was inserted following digestion with EcoRI and SalI enzymes and three versions of 3'UTR (UTR-1, UTR-2, and UTR-3) were inserted following digestion with BamHI and ApaI enzymes. The used primers are shown in Table 8. The EGFP coding sequence was obtained from pEGFP-C1, and 5'UTR and 3'UTR of CTNNB1 were obtained by synthesizing cDNA from RNA extracted from the SW480 cell line, followed by PCR.

FIG. 8

| **Primer** | **Restrict ion enzyme** | **Sequence (5'→3')** | **SEQ ID NO** |
|---|---|---|---|
| GFP Forward primer | SalI | CCCGTCGACTTACTTGTAC | 65 |
| | | AGCTCGTCCATGCC | |
| GFP Reverse primer | BamHI | CCCGGATCCATGGTGAGCA AGGGCGAG | 66 |
| 5'UTR Forward primer | EcoRI | CCCGAATTCAGGATACAGC GGCTTCTGCG | 67 |
| 5'UTR Reverse primer | SalI | CCCGTCGACTGTCCACGCT GGATTTTCAA | 68 |
| UTR-1 Forward primer | BamHI | CCCGGATCCGAGTAACAAT ACAAATGGATTTTG | 69 |
| UTR-1 Reverse primer | ApaI | CGCGGGCCCCAATCGAATG AATTAAAAGTTTA | 70 |
| UTR-2 Forward primer | BamHI | CCCGGATCCGTAACTGTTT TTTAAGTCTCTCGT | 71 |
| UTR-2 Reverse primer | ApaI | CGCGGGCCCACACCTCTTA CTGATTTACCC | 72 |

T7 polymerase protein was purified by the following method. Clones obtained by transforming the His-T7 RNA polymerase expression vector into the BL21 cell line were incubated in 500 mL of liquid LB medium at 37°C overnight. When theOD₆₀₀ value reached 0.4, 0.5 mM IPTG was added, followed by incubation again for 3 hours. After cell down, the pellets were resuspended in a lysis buffer (20 mM Tris-Cl (pH 8.0), 0.1% Triton X-100, 200 mM NaCl, 1 mM Imidazole). The resuspension was incubated on ice for 20 minutes and then sonicated, followed by amplification at 60% for 15 seconds, repeated for 20 cycles. The supernatant obtained by centrifugation at 23,000 × g for 30 minutes at 4°C was precleared twice with a lysis buffer, followed by shaking with 1.5 mL of His-resin at 4°C for 4 hours. The supernatant was discarded through centrifugation to obtain only a resin, and then an elution buffer (20 mM Tris-Cl (pH 8.0), 0.1% Triton X-100, 200 mM NaCl, 200 mM Imidazole) 6 times the volume of the resin was added, followed by incubation at 4°C overnight. After centrifugation, only the supernatant was obtained, and only T7 RNA polymerase was concentrated using AmiconUltra-2 Centrifugal Filter Unit (Merk, UFC205024). The obtained T7 RNA polymerase was placed in a storage buffer (50 mM Tris-Cl (pH 7.9), 100 mM NaCl, 20 mM DTT, 1 mM EDTA, 50% glycerol, 0.1% Triton X-100), aliquoted into 200 µL portions, and stored at -20°C.

*In vitro* transcription was performed as follows. 10× T7 polymerase buffer (200 mM HEPES-KOH (pH 7.5), 10 mM MgCl2, 40 mM DTT, 2 mM spermidine), 10 mM rNTP, 20 U RNase inhibitor, and 1 µL of purified T7 RNA polymerase were added and incubated at 37°C overnight. The synthesized RNA was loaded onto an 8% urea-acrylamide gel, and only RNA fragments with an appropriate size were cleaved and subjected to phenol/chloroform precipitation and then purified with the RNA Clean & Concentrator (Zymo).

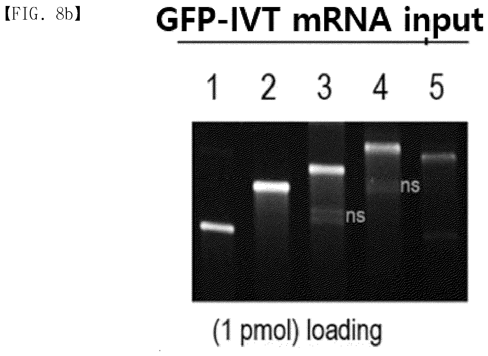

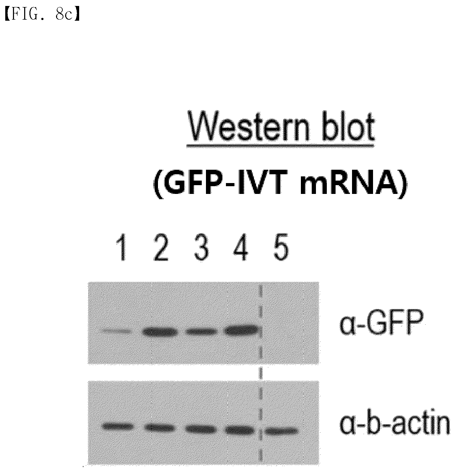

Each IVT-RNA was loaded onto a TBE agarose gel to confirm RNA production (FIG. 8B). IVT-RNA was transfected into U2OS cells, and protein expression efficiency was analyzed by western blotting. As a result, protein expression efficiency was higher for β-catenin UTR RNA than β-actin 3'UTR (FIG. 8C).

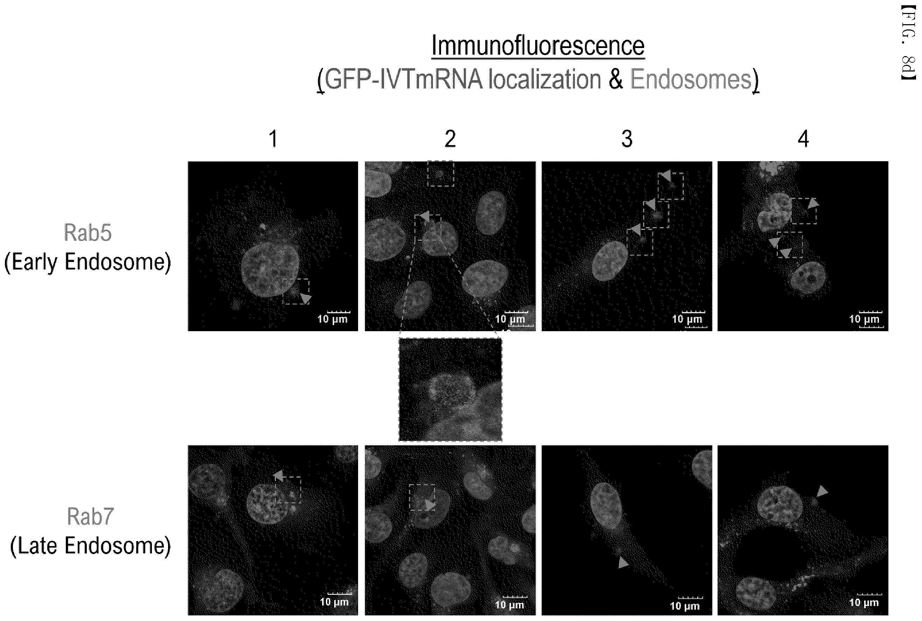

The RNA was transfected into U2OS cells, and GFP and the endosome markers Rab5 (a marker of early endosome) and Rab7 (a marker of late endosome) were observed through immunofluorescence analysis. As shown in FIG. 8D, when only the GFP coding region was present or when 5'UTR RNA was added, the protein was aggregated in the form of an aggresome near the nucleus and trapped in the early endosome. However, for UTR-1 and UTR-2 RNA, the protein was expressed in the form of granules in the cytoplasm, expressed far away, up to the vicinity of the cell membrane, and showed a pattern of escaping the late endosome (FIG. 8D).

Therefore, it was confirmed that even when using of IVT-RNA, β-catenin UTR can enhance protein expression and promote the movement of proteins to the cytoplasm or cell membrane, suggesting that β-catenin UTR can be utilized as an RNA vaccine structure.

### Example 7-2. Analysis of localization of in vitro-RNA depending on the presence or absence of β-catenin 3'UTR fragments by using luciferase

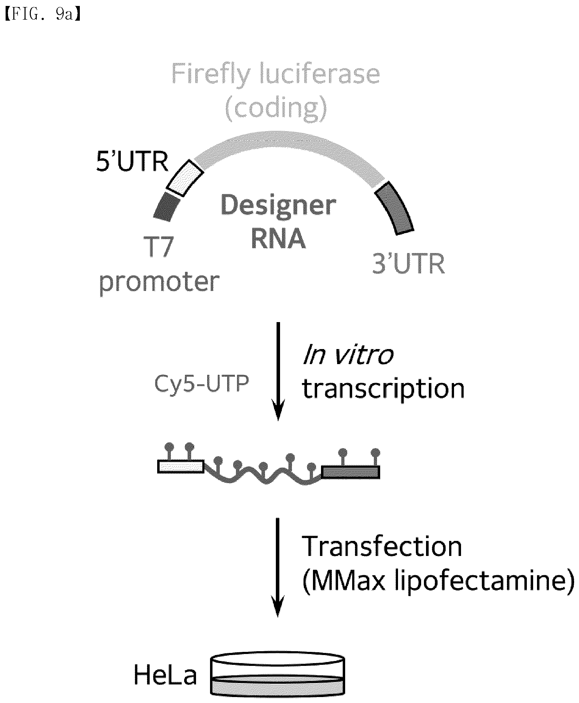

To analyze whether β-catenin 3'UTR fragments affect the intracellular expression pattern of *in vitro*-RNA (IVT-RNA), IVT-RNA-luciferase constructs were constructed (FIG. 9A).

PCR was performed to obtain dsDNA template comprising T7 promoter sequence (SEQ ID NO: 75, TAATACGACTCACTATAGGG). The used primers are shown in Table 9 below.

**Table 9**

| **Primer** | **Sequence (5'→3')** | **SEQ ID NO** |
|---|---|---|
| T7 Luciferase Forward primer | AAATAATACGACTCACTATAGGGAG GATACAGCGGCTTCTGCG | 74 |
| T7 Luciferase Reverse primer | CGGAAAGATCGCCGTGTAA | 75 |
| T7 Luciferase 3'UTR F1 Reverse primer | GAGAGACTTAAAAAACAGTTACTGC | 76 |
| T7 Luciferase 3'UTR F1+F3 Reverse primer | ACACCTCTTACTGATTTACCCTA | 77 |

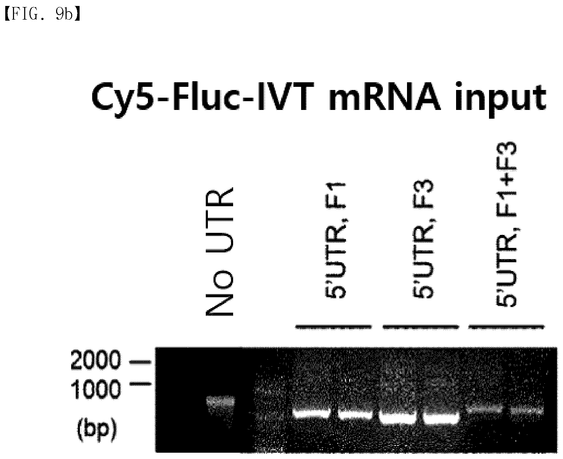

The PCR products thus obtained were purified using the Gel and PCR Clean-up Kit (LaboPass #CMA0112). Each IVT-RNA was loaded onto a TBE agarose gel, thereby confirming the production of RNA (FIG. 9B).

The *in vitro* transcribed RNA was synthesized by the HiScribe^{®} T7 High Yield RNA Synthesis Kit (NEB # E2040S) using 50 ng of each PCR product as a template. To reveal the intracellular localization of the transfected RNA, UTP with cyanine 5 (Cy5) fluorescence was additionally used. An experimental composition was as follows: 1X Reaction Buffer, 10 mM ATP, 10 mM CTP, 10 mM GTP, 7.5 mM UTP, 2.5 mM Cy5-UTP, 2 µl T7 RNA Polymerase Mix. The experimental composition was placed in a 200-µl PCR tube and incubated at 37°C for 4 hours in an air chamber incubator. To remove the PCR template inserted as template DNA, the TURBO DNA-free^{™} Kit (Invitrogen #AM1907) was used according to the manual. The samples that had been inactivated by DNase I were purified using the MEGAclear^{™} Transcription Clean-Up Kit, and 100 µg to 250 µg of purified RNAs were obtained for the samples.

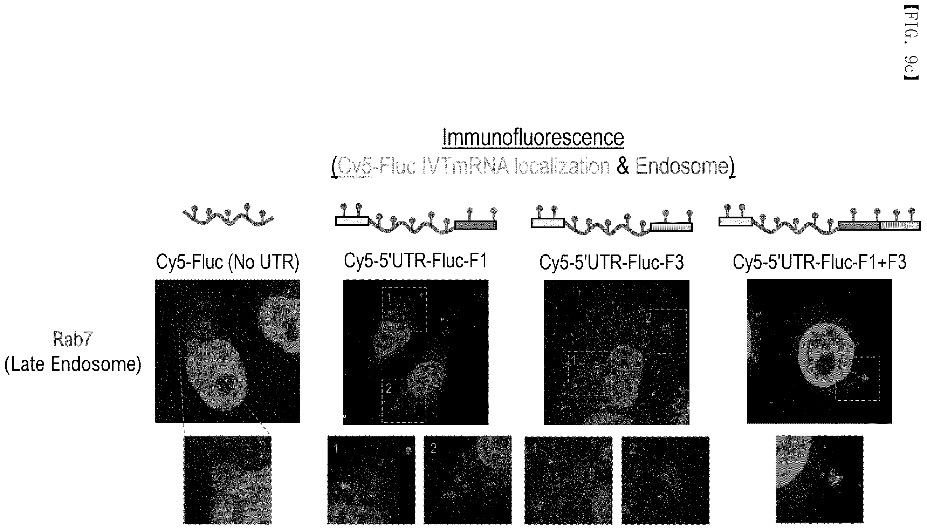

The HeLa cells were transfected with *in vitro* transcribed RNA using the Lipofectamine^{™} MessensgerMAX^{™} (Invitrogen #LMRNA015) according to the manufacturer's protocol. The amount of transfection was 0.5 pmol in an 8-well dish, and after 24 hours of transfection, the cells were washed three times with 1× PBS buffer and then fixed with 3.7% paraformaldehyde for 5 minutes. The cells were washed three times with 1× PBS buffer, incubated with 0.5% Triton X-100 for 5 minutes for cell permeabilization, and washed three times with 1× PBS buffer. To investigate co-localization with the late endosome marker Rab7, anti-Rab7 antibody (Abcam #ab137029) diluted 1:500 was incubated at 4°C overnight. The cells were washed three times with 1× PBS buffer, and then anti-rabbit IgG-Alexa 488 antibody (Invitrogen #A-21206) was incubated at room temperature for 2 hours. The cells were washed three times with 1× PBS buffer and then mounted using the In Situ Mounting medium with DAPI, and the images thereof were taken using a confocal microscope. The taken images are shown in FIG. 9C.

As shown in FIG. 9C, the absence of β-catenin 3'UTR fragments caused the protein to be trapped in the endosome, but the presence of β-catenin 3'UTR fragments showed a pattern of escaping the late endosome.

It can be therefore identified that even in using of IVT-RNA, β-catenin 3'UTR fragments, especially, Fragment 1, Fragment 3, and a combination thereof can improve protein expression and promote the movement of proteins to the cytoplasm or cell membrane, and thus such a fragment can be utilized as an RNA vaccine structure.

Although the present invention has been described in detail with reference to the specific features, it will be apparent to those skilled in the art that this description is only for a preferred embodiment and does not limit the scope of the present invention.

## Claims

1. An mRNA molecule comprising:
(a) a coding region encoding a target protein;
(b) a 5'untranslated region (UTR) of β-catenin attached to the 5'-end of the coding region; and
(c) a 3'UTR of β-catenin or a fragment thereof attached to the 3'-end of the coding region.

2. The mRNA molecule of claim 1, wherein the fragment of the 3'UTR has a deletion of at least an intron 15 (In15) site in the full-length 3'UTR sequence.

3. The mRNA molecule of claim 2, wherein the fragment of the 3'UTR further has a deletion of an exon 16A (E16A) site.

4. The mRNA molecule of claim 1, wherein the fragment of the 3'UTR comprises an RNA sequence selected from the group consisting of the RNA sequence of SEQ ID NO: 6, the RNA sequence of SEQ ID NO: 7, the RNA sequence of SEQ ID NO: 9, the RNA sequence of SEQ ID NO: 10, the RNA sequence of SEQ ID NO: 11, the RNA sequence of SEQ ID NO: 12, the RNA sequence of SEQ ID NO: 13, the RNA sequence of SEQ ID NO: 14, the RNA sequence of SEQ ID NO: 15, and a combination thereof.

5. The mRNA molecule of claim 1, wherein the fragment of the 3'UTR comprises an RNA sequence selected from the group consisting of the RNA sequence of SEQ ID NO: 9, the RNA sequence of SEQ ID NO: 11, the RNA sequence of SEQ ID NO: 13, and a combination thereof.

6. The mRNA molecule of claim 1, wherein the 5'UTR comprises the RNA sequence of SEQ ID NO: 1.

7. A nucleic acid molecule encoding the mRNA molecule of any one of claims 1 to 6.

8. An expression construct comprising the nucleic acid molecule of claim 7.

9. A recombinant vector comprising the expression construct of claim 8.

10. An isolated host cell comprising the recombinant vector of claim 9.

11. A method for preparing an mRNA molecule to improve the expression efficiency of a target protein and promote the movement of the target protein into the cytoplasm or cell membrane, the method comprising attaching a 5'-UTR of β-catenin and a 3'UTR of β-catenin or a fragment thereof to the 5'-end and 3'-end of an RNA sequence encoding the target protein, respectively.

12. The method of claim 11, wherein the fragment of the 3'UTR has a deletion of at least an intron 15 (In15) site in the full-length 3'UTR sequence.

13. The method of claim 12, wherein the fragment of the 3'UTR further has a deletion of an exon 16A (E16A).

14. The method of claim 11, wherein the fragment of the 3'UTR comprises an RNA sequence selected from the group consisting of the RNA sequence of SEQ ID NO: 6, the RNA sequence of SEQ ID NO: 7, the RNA sequence of SEQ ID NO: 9, the RNA sequence of SEQ ID NO: 10, the RNA sequence of SEQ ID NO: 11, the RNA sequence of SEQ ID NO: 12, the RNA sequence of SEQ ID NO: 13, the RNA sequence of SEQ ID NO: 14, the RNA sequence of SEQ ID NO: 15, and a combination thereof.

15. The method of claim 11, wherein the fragment of the 3'UTR comprises an RNA sequence selected from the group consisting of the RNA sequence of SEQ ID NO: 9, the RNA sequence of SEQ ID NO: 11, the RNA sequence of SEQ ID NO: 13, and a combination thereof.

16. The method of claim 11, wherein the 5'UTR comprises the RNA sequence of SEQ ID NO: 1.

17. The method of claim 11, wherein the mRNA molecule is prepared through *in vitro* transcription.
